# EUROPEAN PATENT SPECIFICATION

(11) **EP 3 463 291 B1**
(45) Date of publication and mention of the grant of the patent: **29.07.2026**
(21) Application number: 17740088.4
(22) Date of filing: 31.05.2017
(51) Int. Cl.: A61K 9/06, A61K 47/10, A61K 31/365, A61K 31/4045, A61K 31/4375, A61K 31/4741, A61K 31/4748, A61K 31/7048, A61K 35/745, A61K 35/747, A61K 45/06, A61P 17/00, A61P 17/06, A61P 37/02, A61P 37/08

(54) **BACTERIA-BASED GEL COMPOSITIONS FOR TOPICAL APPLICATIONS AND USES THEREOF**
BAKTERIENBASIERTE GELZUSAMMENSETZUNGEN ZUR TOPISCHEN ANWENDUNGEN UND VERWENDUNGEN DAVON
COMPOSITIONS SOUS FORME DE GEL À BASE DE BACTÉRIES POUR APPLICATIONS TOPIQUES ET UTILISATIONS ASSOCIÉES

(30) Priority: 01.06.2016 IT UA20164065
(43) Date of publication of application: 10.04.2019
(73) Proprietor: Probiotical S.p.A., 28100 Novara (IT)
(72) Inventor: MOGNA, Giovanni, 28100 Novara (IT)
(74) Representative: Benedetto, Marco
(86) International application number: PCT/IB2017/053206
(87) International publication number: WO 2017/208172

(56) References cited:
- EP-A1- 1 769 821
- WO-A1-02/074325
- WO-A1-2008/058755
- WO-A1-2010/062153
- WO-A2-00/71139
- WO-A2-2006/013441
- DE-A1- 10 154 324
- US-A1- 2014 243 384
- AROONSRI PRIPREM ET AL: "Effect of Polymeric Combinations on Mucoadhesive and Swelling Properties of Orabase Gel Formulations", ADVANCED MATERIALS RESEARCH, vol. 853, 24 December 2013 (2013-12-24), pages 3 - 8, XP055338521, DOI: 10.4028/www.scientific.net/AMR.853.3

## Description

The present invention relates to a composition for use as a medicament in the form of a gel with characteristics of a reversible thermogel, based on a mixture of poloxamers, water and optionally excipients, and an effective amount of an active substance. Furthermore, the present invention relates to a process for the preparation of said composition.

In the market, there exist various forms of administration of active ingredients, e.g. microorganisms such as bacteria, at least one flavonoid such as rutin, oxerutin, diosmin, hesperidin and troxerutin, chemotherapeutic agents and/or arctigenin, arctin, berberine, berbamine, sanguinarine and chelerythrine, or rutoxides, as such or in the form of plant extracts containing said compounds, or melatonin or derivatives thereof. The topical forms of administration can be patches, creams, gels or suspensions. An example of a gel composition is described in EP2520279 A1. WO2006013441 discloses the use of probiotical bacteria for the preparation of a topical composition for the protection of the skin from the microbical diseases, in particular for preventing and/or treating the microbical diseases of the skin. Preferred probiotical bacteria are those belonging to the genus *Lactobacillus.* WO2006013441 discloses that a probiotical bacterium particularly advantageous *L. plantarum* P 17630 (commercialized under trademark Bactocin^{®}) and that other advantageous probiotical bacteria are, for example, *L. gasseri* P 18137, *L. gasseri* P17632 and the *L. crispatus* P 17631.

However, there continues to be a felt need to be able to have a form of administration for topical use for transdermal application that is economical and easy to prepare, stable, efficacious in dosing the effective amount of active substance contained therein, and easily absorbable at the level of the cutis, dermis and epidermis without leaving residues or streaks.

The Applicant has developed a composition in gel form for topical use which provides an adequate and advantageous response to the above-mentioned needs.

The present invention relates to a composition in the form of a gel, wherein said composition is for topical transdermal use in the preventive and/or curative treatment of pathologies, disorders or diseases associated with/deriving from alterations of the immune system selected from the group comprising allergies, atopy, food hypersensitivity, dermatitis, atopic dermatitis, eczema and psoriasis, wherein the composition has the features as claimed in the accompanying claims.

The present invention relates to a process for the preparation of said composition for use, having the features as claimed in the accompanying claims.

The invention is defined by the claims. Any subject-matter falling outside the scope of the claims is provided for information purposes only. The preferred embodiments of the present invention set forth in the description that follows are illustrated solely by way of example and in no way limit the broad scope of application of the present invention, which will appear clear to the person skilled in the art.

In the context of the present invention, composition(s) means a pharmaceutical composition or a composition for medical devices.

Advantageously, the composition of the present invention is capable of releasing the active substance or active ingredient over time, in a constant, gradual and lasting manner as a controlled transdermal release, in such a way as to prolong its activity and therapeutic effectiveness over time.

Advantageously, the composition of the present invention is a gel in the form of a reversible thermogel thanks to the presence of a vehicle or carrier which contains specific selected polymers of the poloxamer type, as presently claimed.

Advantageously, the composition of the present invention is for topical transdermal use, said composition is furthermore economical and easy to prepare, stable, efficacious in dosing the effective amount of active substance contained therein over time, and easily absorbable at the level of the cutis, dermis and epidermis without leaving residues or streaks.

The present invention relates to a composition (abbreviated as CMP1) in the form of a gel for topical use. Said composition comprises:
- an effective amount of an active substance, and
- a vehicle or carrier comprising water and a thickening viscous matrix comprising or, alternatively, consisting of a mixture of Poloxamer 188 and Poloxamer 407, wherein Poloxamer 188 is present in an amount comprised from 0.1% to 5% by weight, out of 100 g of composition, preferably in an amount comprised from 0.5% to 5% by weight or from 1% to 3% by weight; and Poloxamer 407 is present in an amount comprised from 1% to 30% by weight, out of 100 g of composition, preferably in an amount comprised from 5% to 30% by weight or from 10% to 20% by weight; wherein the active substance is at least one microorganism selected from the group comprising or, alternatively, consisting of the strains *Lactobacillus salivarius* (LS01) DSM 22775, deposited on 23.07.2009; *Bifidobacterium breve* (BR03) DSM 16604, deposited on 20.07.2004; *Lactobacillus pentosus* (LPS01) DSM 21980, deposited on 14.11.2008; *Streptococcus thermophilus* (FP4) DSM 18616, deposited on 13.09.2006; *Lactobacillus casei ssp. rhamnosus* (LR04) DSM 16605, deposited on 20.07.2004; and *Lactobacillus acidophilus* (LA02) DSM 21717, deposited on 06.08.2008, and a mixture thereof;
preferably the strain is *Lactobacillus salivarius* (LS01) DSM 22775, and/or *Bifidobacterium breve* (BR03) DSM 16604, and/or *L pentosus* (LPS01) DSM 21980, and wherein said composition is for topical transdermal use in the preventive and/or curative treatment of pathologies, disorders or diseases associated with/deriving from alterations of the immune system selected from the group comprising allergies, atopy, food hypersensitivity, dermatitis, atopic dermatitis, eczema and psoriasis. Said vehicle or carrier comprises water, preferably purified water, until reaching 100% by weight of the composition. The thickening viscous matrix optionally comprises additives and/or excipients and/or adjuvants. Said additives and/or excipients and/or adjuvants are selected from among those capable of promoting the formation and stabilisation of the gel (reversible thermogel) and are selected from the group comprising or, alternatively, consisting of acid salts, sodium sorbate, potassium sorbate, sodium benzoate, potassium benzoate, glycols, ethylene glycol and propylene glycol.

Advantageously and according to the invention, the composition CMP1 contains Poloxamer 188 (Lutrol F66) CAS No. 9003-11-6 with an average molecular weight of 7680-9510 and Poloxamer 407 (Lutrol F127 Prill) CAS No. 900-11-6 with an average molecular weight of 984014600.

In another preferred embodiment (CMP4), the composition CMP1 comprises said microorganisms at a concentration comprised from 1x10⁶ CFU/g to 1x10¹² CFU/g of composition, preferably from 1x10⁷ CFU/g to 1x10¹¹ CFU/g of composition, even more preferably from 1x10⁸ CFU/g to 1x10¹⁰ CFU/g of composition, for example 1x10⁹ CFU/g of composition, and wherein:
- said microorganisms are present in said composition in an amount by weight comprised from 0.1% to 5%, preferably in an amount by weight comprised from 0.5% to 3%, even more preferably in an amount by weight comprised from 1% to 2%, relative to the total weight of the composition (CMP4).

In another preferred embodiment (CMP5), the composition CMP1 or 4 comprises an effective amount of ar active substance selected from:
(iii) at least one compound selected from the group comprising or, alternatively, consisting of rutin, rutoxides, arbutin, oxerutin, diosmin, hesperidin and troxerutin, arctigenin, arctin, berberine, berbamine, sanguinarine and chelerythrine, as such or in the form of plant extracts containing said compounds, on their own; and
(ii) melatonin;
said active substance being present in said composition in an amount by weight comprised from 0.1% to 10% by weight preferably in an amount comprised from 0.5% to 5% by weight even more preferably from 1% to 3% by weight relative to the total weight of the composition (CMP5).

The present invention relates to a process for the preparation of a composition in accordance with the compositions CMP1 or 4 or 5, said process comprising the following steps:
- mixing in water, under continuous stirring, the various components, added in succession one after the other or separately premixed and added together, using a mixer provided with a temperature control and adjustment means and a stirring means, wherein the temperature is set at values tower than room temperature, comprised from 2°C to 16°C, preferably from 4°C to 10°C, for example from 6°C to 8°C, for a time comprised from 1 minute to 15 minutes so as to obtain a homogeneous mixture;
- maintaining said homogeneous mixture, preferably under continuous stirring, at a temperature comprised from 2°C to 10°C, preferably at a temperature comprised from 4°C to 8°C, for example from 5°C to 7°C, for a time comprised from 1 minute to 60 minutes, preferably from 5 minutes to 40 minutes, even more preferably from 10 minutes to 20 minutes, so as to obtain said composition in a liquid state;
- packaging said composition in sealed containers.

The vehicle present in the composition of the present invention comprises water and a thickening viscous matrix according to the claims. In the context of the present invention, the term *"reversible thermogel*" refers to a gel obtained from aqueous copolymer solutions, wherein the copolymer is a poloxamer, capable of gelling in a reversible manner according to the temperature.

Typically, the composition, i.e. the reversible thermogel, of the present invention is liquid at temperatures below room temperature and becomes gelatinous at temperatures close to human body temperature. For example, said composition is liquid at temperatures approximately comprised from 3°C to 15°C; in particular, at about 5°C. Said composition in turn gels, that is, becomes a gel, starting from about 20°C until about 37°; in particular it gels between about 20° and 25°C, preferably, between about 21° and 23°C.

In the context of the present invention, the active agent, for example the melatonin, is present In the composition of the invention in an amount comprised from 0.1% to 3% by weight out of 100 g of composition; preferably, comprised from 0.5% to 2% by weight, out of 100 g of composition; more preferably, about 1% by weight, out of 100 g of composition; even more preferably, 1% by weight, out of 100 g of composition. The melatonin can be added into the composition as such, in powder, with a purity comprised from 96% to 99.9%; in particular, with a purity comprised from 97% to 99%; for example, with a purity of the 98%. Alternatively, the melatonin can be added into the composition also in the form of microparticles, for example of a size comprised from 50 to 100 µm.

In the context of the present invention, the poloxamers are a series of block copolymers of ethylene oxide and propylene oxide in accordance with the following structural formula:

HO(C₂H₄O)ₐ(C₃H₆O)_{b}(C₂H₄O)ₐH,

where a and b are whole numbers expressing the number of the oxyethylene and oxypropylene residues present in the molecule. Poloxamers are non-ionic copolymers of polyoxyethylene-polyoxypropylene used primarily in pharmaceutical formulations as emulsifying, solubilising or wetting agents.

The poloxamers of the present invention are capable of thickening the aqueous solutions and giving rise to reversible thermogels possessing rheological properties that vary according to their concentration and molecular weight.

According to the invention, a mixture of Poloxamer 188 and Poloxamer 407 is used.

In a preferred embodiment of the invention,
the Poloxamer 188 is present in an amount comprised from 1% to 5% by weight out of 100 g of composition; preferably, comprised from 1% to 3% by weight, out of 100 g of composition; more preferably, comprised from 1% to 2% by weight out of 100 g of composition; even more preferably, of 1% by weight out of 100 g of composition; and
the Poloxamer 407 is present in an amount comprised from 15% to 30% by weight out of 100 g of composition; preferably, comprised from 18% to 25% by weight out of 100 g of composition; more preferably, comprised from 20% to 23% by weight out of 100 g of composition; even more preferably, of 21% by weight, out of 100 g of composition.

In the context of the present invention, the water is preferably purified water and is present in an amount such as to reach a balance at 100% by weight of the composition.

The composition of the present invention further comprises known additives/excipients/adjuvants commonly used in the pharmaceutical formulation technique; in particular, said additives/excipients/adjuvants are selected from among those particularly advantageous for the formation and the stabilisation of the reversible thermogel of the invention. In a preferred embodiment of the invention, said additives/excipients/adjuvants are selected from the group consisting of acid salts, such as sodium sorbate, potassium sorbate, sodium benzoate, potassium benzoate; and glycols, such as ethylene glycol and propylene glycol.

In a particularly preferred embodiment, said additives/excipients/adjuvants consist of potassium sorbate, sodium benzoate and propylene glycol, in a total amount comprised from 1 to 5% by weight, out of 100 g of composition. The pharmaceutical composition for topical use in the form of a reversible thermogel of the present invention can be prepared by adopting well-known apparatus and processing conditions commonly used in the industry for the preparation of a reversible thermogel.

Essentially, the various ingredients making up the desired composition, added in succession or remixed, undergo cold mixing using a suitable mixer provided with a refrigeration temperature control and adjustment means and a stirring means. The mixing temperature is set on values below room temperature, preferably between 3° and 8°C, and the mixture of components is kept under cold stirring for an amount of time sufficient to obtain a completely homogeneous mixture which, over the course of time, will transform, still cold, into a completely liquid solution.

The product thus obtained is then packaged in suitable containers, e.g. sealed tubes, and subsequently, on reaching room temperature, will transform into a gel, in particular at the moment when it is applied on the skin.

In one embodiment, the present invention relates to a composition as described above wherein said composition further comprises an effective amount of an active substance selected from:
(ii) at least one compound selected from the group comprising or, alternatively, consisting of rutin, rutoxides, arbutin, oxerutin, diosmin, hesperidin and troxerutin, arctigenin, arctin, berberine, berbamine, sanguinarine and chelerythrine, as such or in the form of plant extracts containing said compounds, on their own; and/or
(iii) melatonin;

and, optionally, additives and/or excipients and/or adjuvants selected from among those capable of promoting the formation and stabilisation of the gel, selected from the group comprising or, alternatively, consisting of acid salts, sodium sorbate, potassium sorbate, sodium benzoate, potassium benzoate, glycols, ethylene glycol and propylene glycol;
said composition being for topical transdermal use in the preventive and/or curative treatment of pathologies, disorders or diseases associated with/deriving from alterations of the immune system selected from the group comprising allergies, atopy, food hypersensitivity, dermatitis, atopic dermatitis, eczema and psoriasis.

In the composition of the present invention, Poloxamer 188 is present in an amount comprised from 0.1% to 5% by weight, out of 100g of composition, preferably in an amount comprised from 0.5% to 5% by weight, for example from 1% to 3% by weight; and Poloxamer 407 is present in an amount comprised from 1% to 30% by weight, out of 100g of composition, preferably in an amount comprised from 5% to 30% by weight, for example from 10% to 20% by weight.

Table 1 discloses a list of microorganisms. Only the microorganisms as claimed form part of the present invention, namely the strains; Lactobacillus salivarius (LS01) DSM 22775, deposited on 23.07.2009; *Bifidobacterium breve* (BR03) DSM 16604, deposited on 20.072004; *Lactobacillus pentosus* (LPS01) DSM 21980, deposited on 14.11.2008; Streptococcus thermophilus (FP4) DSM 18616, deposited on 13.09.2006; *Lactobacillus casei ssp. rhamnosus* (LR04) DSM 16605, deposited on 20.07.2004; *and Lactobacillus acidophilus* (LA02) DSM 21717, deposited on 06.08.2008, of which it is preferred to use the strains

*Lactobacillus salivarius* (LS01) DSM 22775, and/or *Bifidobacterium breve* (BR03) DSM 16604, and/or *L. pentosus* (LPS01) DSM 21980. In Table 1, any microorganisms not recited in the claims are mentioned solely for illustrative purposes.

Preferably, said composition comprises microorganisms at a concentration comprised from 1x10⁶CFU/g to1x10¹² CFU/g of composition, preferably from 1x10⁷ CFU/g to 1x10¹¹ CFU/g of composition, even more preferably from 1x10⁸ CFU/g to 1x10¹⁰ CFU/g of composition, for example 1x10⁹ CFU/g of composition, and wherein said microorganisms are present in said composition in an amount by weight comprised from 0.1% to 5%, preferably in an amount by weight comprised from 0.5% to 3%, even more preferably in an amount by weight comprised from 1% to 2%, relative to the total weight of composition.

Preferably, the composition for use as described above further comprises an active substance selected from:
(ii) at least one compound selected from the group comprising or, alternatively, consisting of rutin, rutoxides, arbutin, oxerutin, diosmin, hesperidin and troxerutin, arctigenin, arctin, berberine, berbamine, sanguinarine and chelerythrine, as such or in the form of plant extracts containing said compounds, on their own; and/or
(iii) melatonin;
said active substance being present in said composition in an amount by weight comprised from 0.1% to 10% by weight, preferably in an amount comprised from 0.5% to 5% by weight, even more preferably from 1 % to 3% by weight relative to the total weight of the composition.

Solely by way of example, which does not limit the various possibilities of production, an example of preparation of a reversible thermogel/composition of the present invention is described here below.

### Example 1 - Preparation of a lot of a composition (in the form of a reversible thermogel) of the present invention

| **Component** | **weight (g)** | **%** |
|---|---|---|
| Mixture of bacteria (1) or (2) or (3) or (4) or (5) | 2.00 | 1.00 |
| Poloxamer 188 | 4.00 | 2.00 |
| Poloxamer 407 | 40.00 | 20.00 |
| K sorbate | 0.40 | 0.20 |
| Na benzoate | 1.00 | 0.50 |
| Propylene glycol | 2.00 | 1.00 |
| Purified water | 150.60 | 75.30 |
| **TOTAL** | **200.00** | 100.00 |

Mixture (1) consisting of only *Lactobacillus salivarius* (LS01) DSM 22775, or only *Bifidobacterium breve* (BR03) DSM 16604, or only *L pentosus* (LPS01) DSM 21980.

Mixture (2) consisting of *Lactobacillus salivarius* (LS01) DSM 22775, *Bifidobacterium breve* (BR03) DSM 16604 and *L pentosus* (LPS01) DSM 21980, in a ratio by weight of 1:1:1.

Mixture (3) consisting of *Lactobacillus salivarius* (LS01) DSM 22775 and *Bifidobacterium breve* (BR03) DSM 16604 in a ratio by weight of 1:1.

Mixture (4) consisting of *Lactobacillus salivarius* (LS01) DSM 22775 and *L pentosus* (LPS01) DSM 21980, in a ratio by weight of 1:1.

Mixture (5) consisting of *Bifidobacterium breve* (BR03) DSM 16604 and *L pentosus* (LPS01) DSM 21980, in a ratio by weight of 1:1.

The cold purified water (at 5°C) is weighed into a glass container (for example a beaker) with magnetic stirring, a thermometer, and an external ice bath and the potassium sorbate and sodium benzoate are added under stirring. The mixture is maintained under stirring at 5°C until complete dissolution of the salts. Then the following are added, again under stirring: the two poloxamers (crystalline), in succession or in a mixture, the methionine (in powder, or in the form of micro-/nanoparticles) and the propylene glycol and cold stirring is continued for at least 15 minutes until a homogeneous appearance is obtained. The container is then placed in a refrigerator at 5°C for at least 24 h, until obtaining complete dissolution of the ingredients.

After this, the resulting product (liquid) is packaged in 18-20 g tubes and sealed pending use/experimentation. On reaching room temperature and in contact with the skin the product transforms completely into a gel, which is rapidly absorbed following transdermal administration. The pH of the liquid thermogel is 7.40. The gelling temperature Is 21°-22°C.

The composition in the form of a thermogel of the present invention, topically administered, in particular on wrists and/or forearms, has shown an excellent cutaneous permeability, triggering a fast onset of the pharmacological action. Furthermore, thanks to the fact of going directly into circulation without passing through the gastrointestinal tract (no metabolism by the liver), it has also shown high blood levels of the active ingredient, for example, melatonin, for a prolonged period of time (slow release). This fact has made it possible to apply low dosages of the drug, thus obtaining the desired advantageous results (modest dosage/high, long-lasting activity) compared to the traditional forms of administration thereof.

For example, a study was conducted on 10 healthy volunteers with different formulations of melatonin, observing the accumulation of melatonin in the saliva over time (it is well known that melatonin spreads passively into saliva via the bloodstream, and its concentration therein represents 24%-33% of its plasma levels, that is to say, the amount of free melatonin not bound to globulins). Formulations of melatonin which are not as claimed are not according to the invention.

The following treatments were administered to the volunteers, randomly divided into 3 groups:
1. treatment with a formulation of melatonin in a reversible thermogel with the vehicle or carrier of the present invention (dosage 1 mg/g, single dose)
2. treatment with a formulation of melatonin in a reversible thermogel with the vehicle or carrier of the present invention (dosage 2 mg/g, single dose)
3. treatment with a formulation of melatonin in cream (dosage 1 mg/g, single dose)
4. treatment with melatonin in tablets (dosage 1 mg/tablet).

A saliva sample was taken at time 0 (basal) and at subsequent times (up to 7 h) after administration, using a Cortisol-Salivette^{®} device (SARSTEDT S.r.I., Verona, IT); the melatonin levels in the saliva were determined by LC-MS (liquid chromatography - mass spectrometry) using a triple quadrupole mass spectrometer (ABSciex QTrap 3200) and substantially following the method of Khan et et al., 2013.

The study showed that the formulations of melatonin in a reversible thermogel with the vehicle or carrier of the present invention possess an excellent thermal permeability, a fast onset of the pharmacological action and a lasting duration thereof compared to the other formulations tested.

The pharmaceutical compositions of the present invention also showed to act effectively on all of the patients treated, whereas the commonly used formulations, such as, for example, oral ones, do not act on all individuals. Furthermore, the pharmaceutical compositions of the present invention have also shown to possess good stability over time.

The pharmaceutical compositions of the present invention are for topical transdermal use.

The pharmaceutical compositions described herein have shown to be advantageously active, or in any case very promising in the treatment of a number of disorders/diseases due to dysfunctions of physiological functions connected with circadian rhythms such as, for example, the sleep-wake balance; in the regulation of blood pressure; in eliminating of free radicals; in interacting with the immune system, for example, in the treatment of inflammatory states, in the treatment of acquired immunodeficiencies and in the treatment of viral and bacterial infectious diseases and cancer.

Furthermore, the compositions described herein have shown to be useful: in the treatment of sleep disorders in all the treated subjects, by favouring the quality of sleep, in particular of REM sleep; in the treatment of the autism and Down syndrome, particularly in children, by favouring their balance; in the treatment of Alzheimer's disease; in the treatment of Parkinson's disease, by improving sleep and tremors; in the treatment of the vision disorders, for example in maculopathy of the retina, via external applications in the vicinity of the eye; in the treatment of the pulmonary manifestations of cystic fibrosis in children, where they provide an anti-inflammatory action; in the treatment of prostate cancer, where they are capable of preventing the propagation thereof; in the treatment of the tumours, as anti-inflammatory, anti-tumour adjuvants. According to the invention, the compositions are for topical transdermal use in the preventive and/or curative treatment of pathologies, disorders or diseases associated with/deriving from alterations of the immune system selected from the group comprising allergies, atopy, food hypersensitivity, dermatitis, atopic dermatitis, eczema and psoriasis.

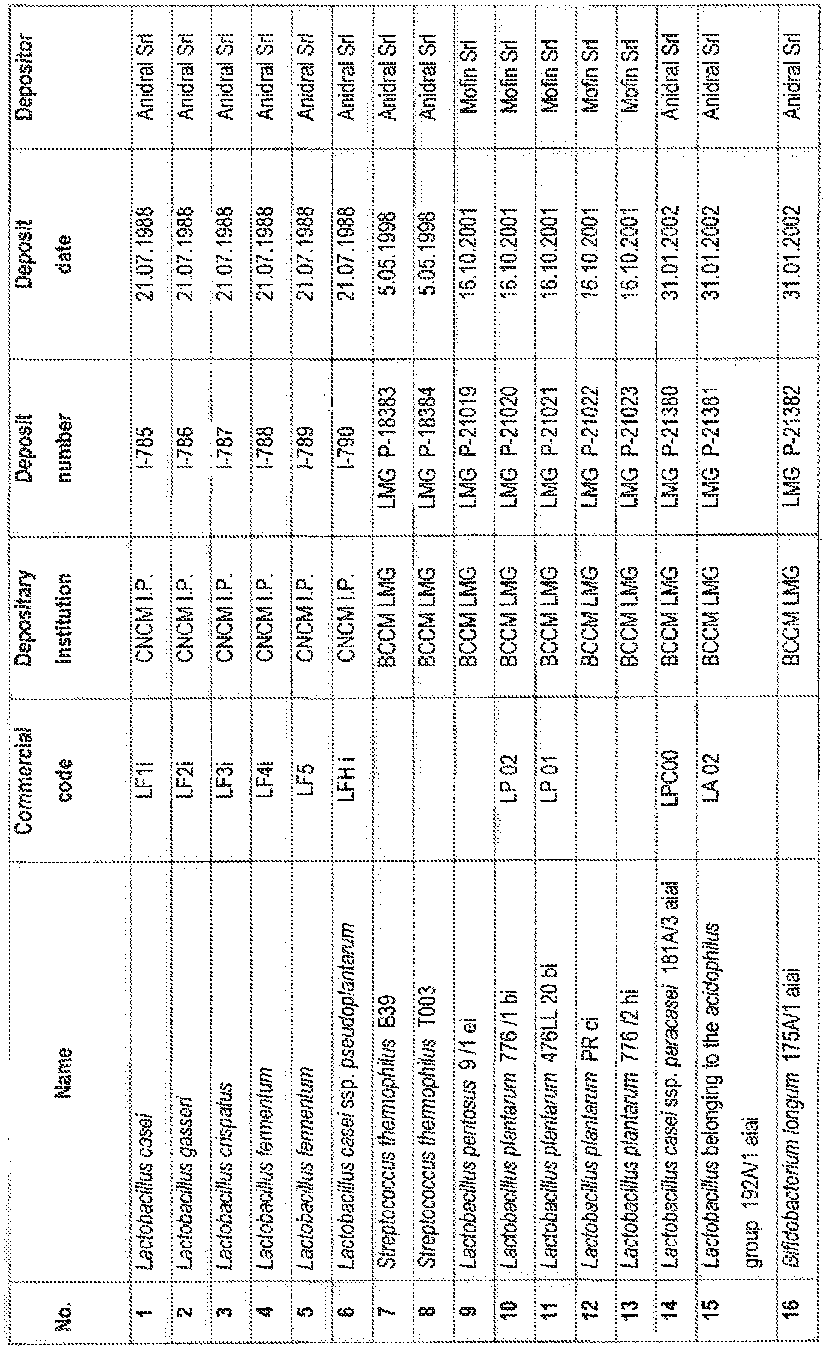

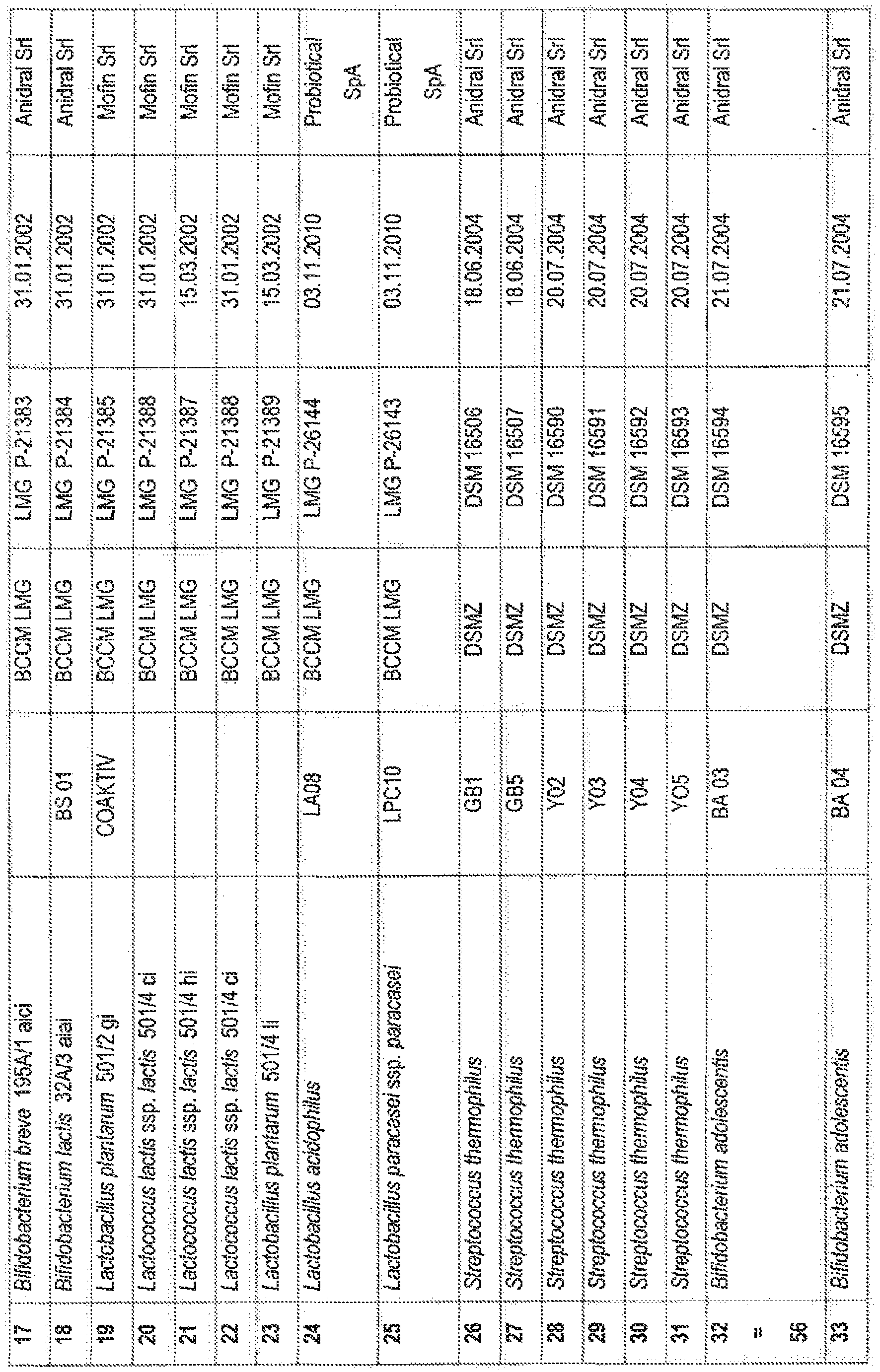

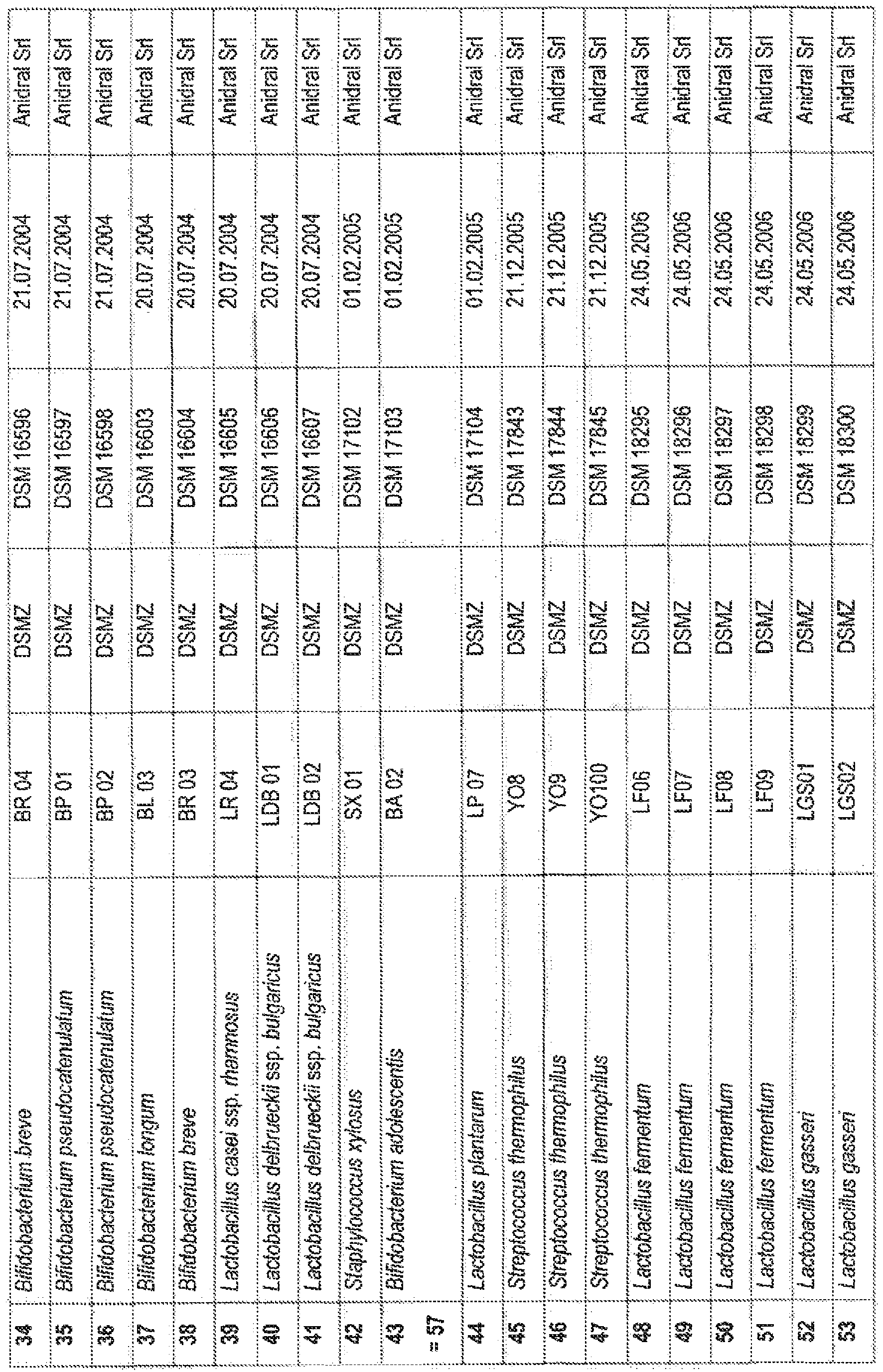

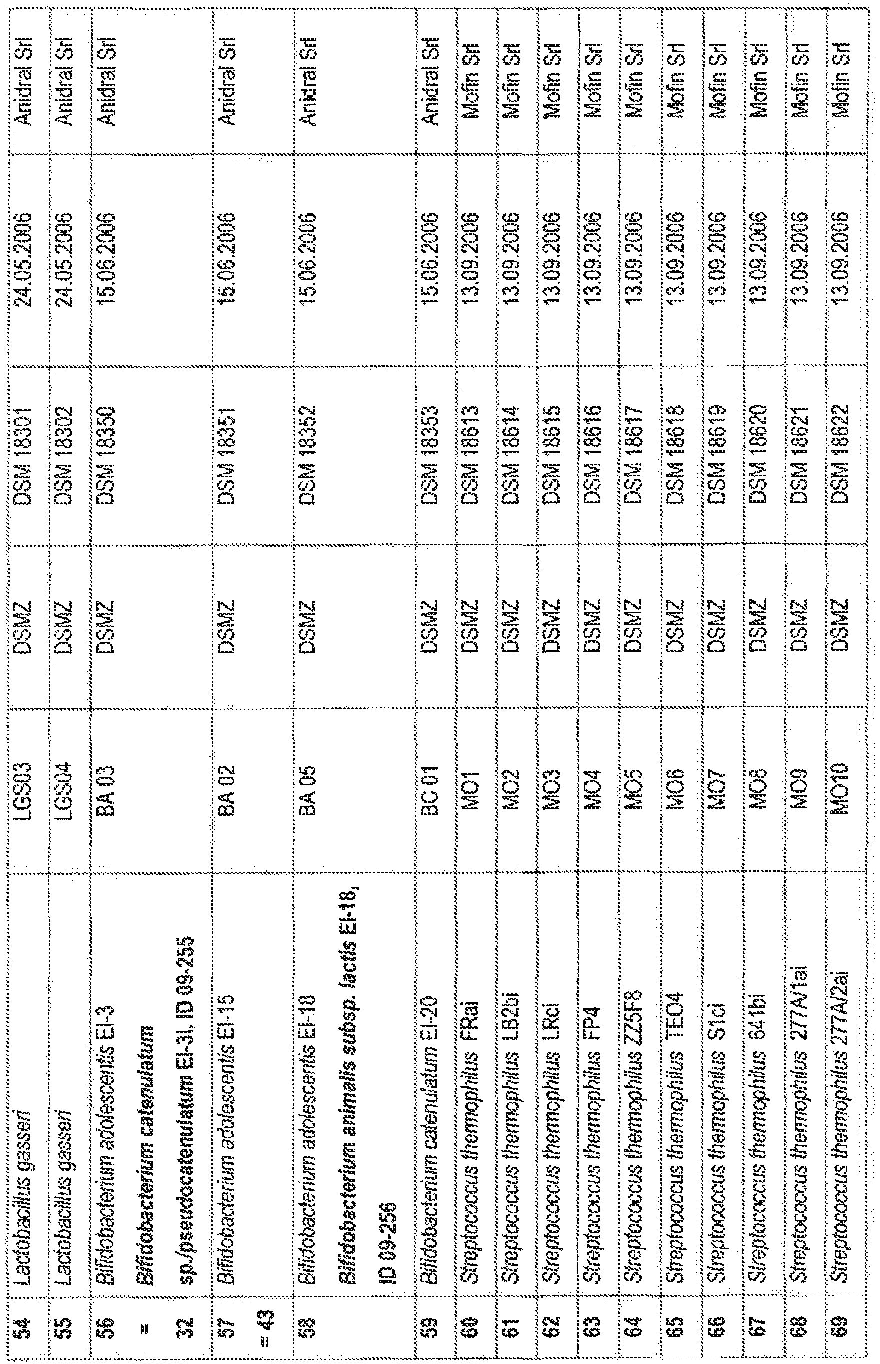

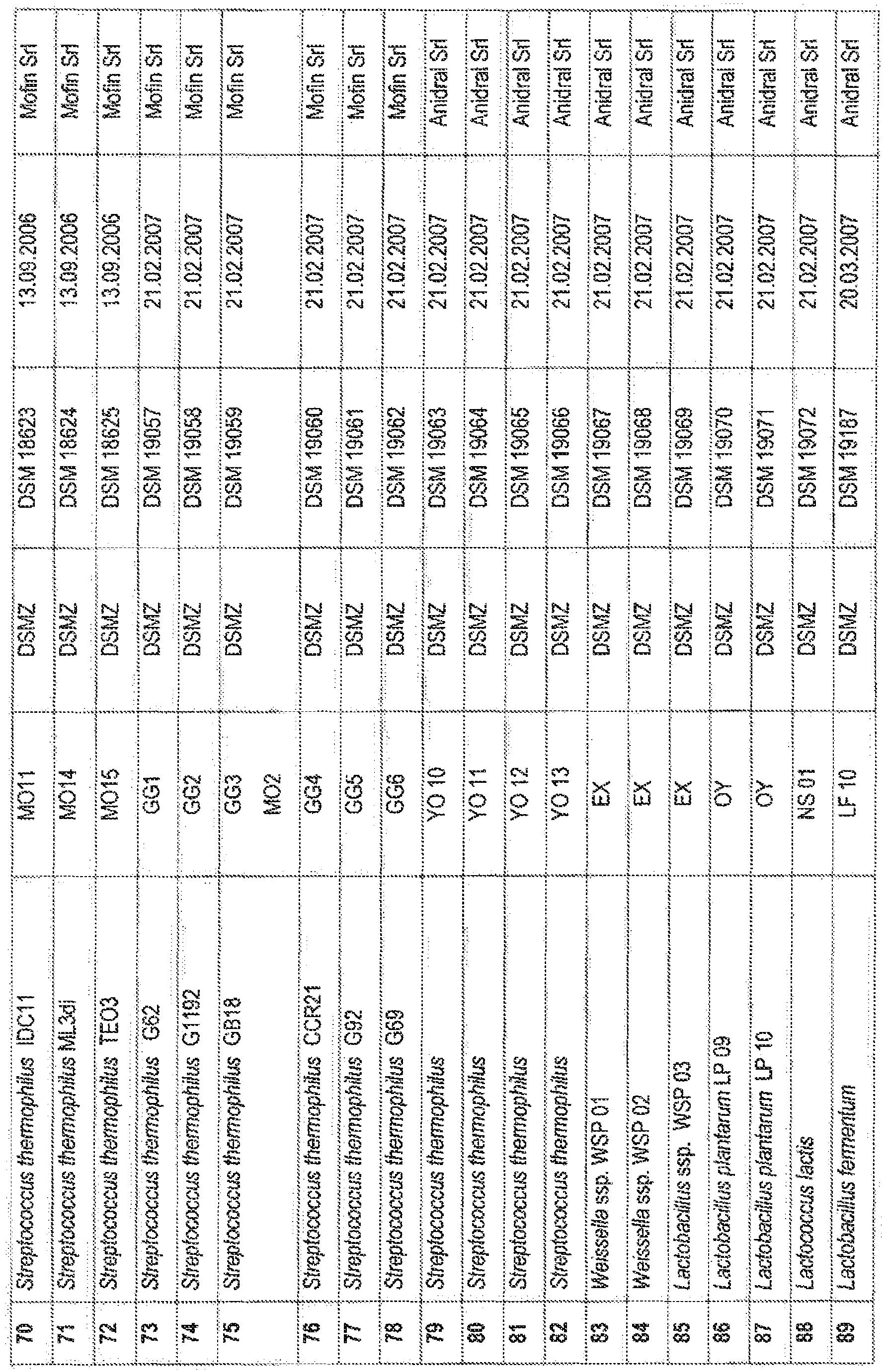

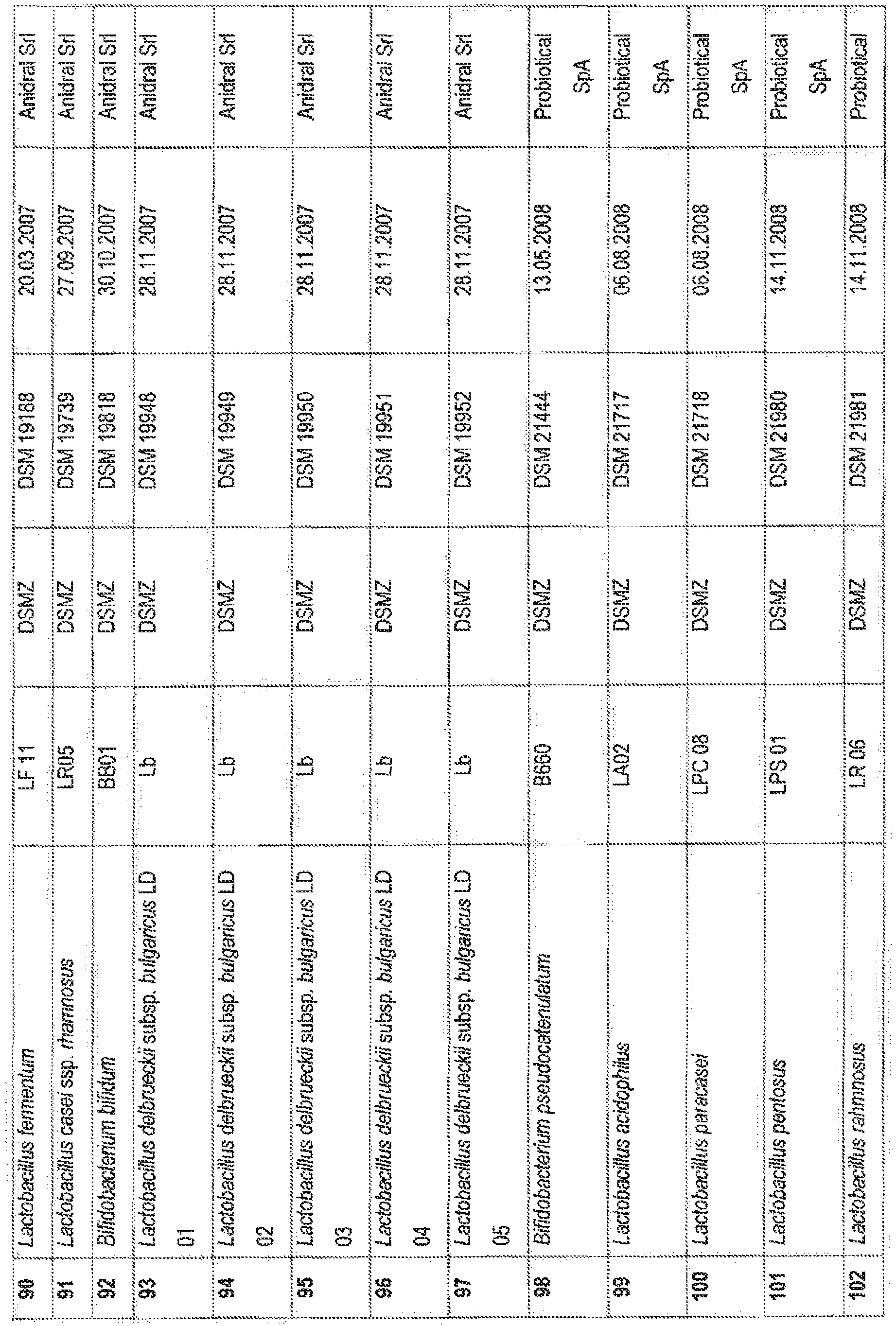

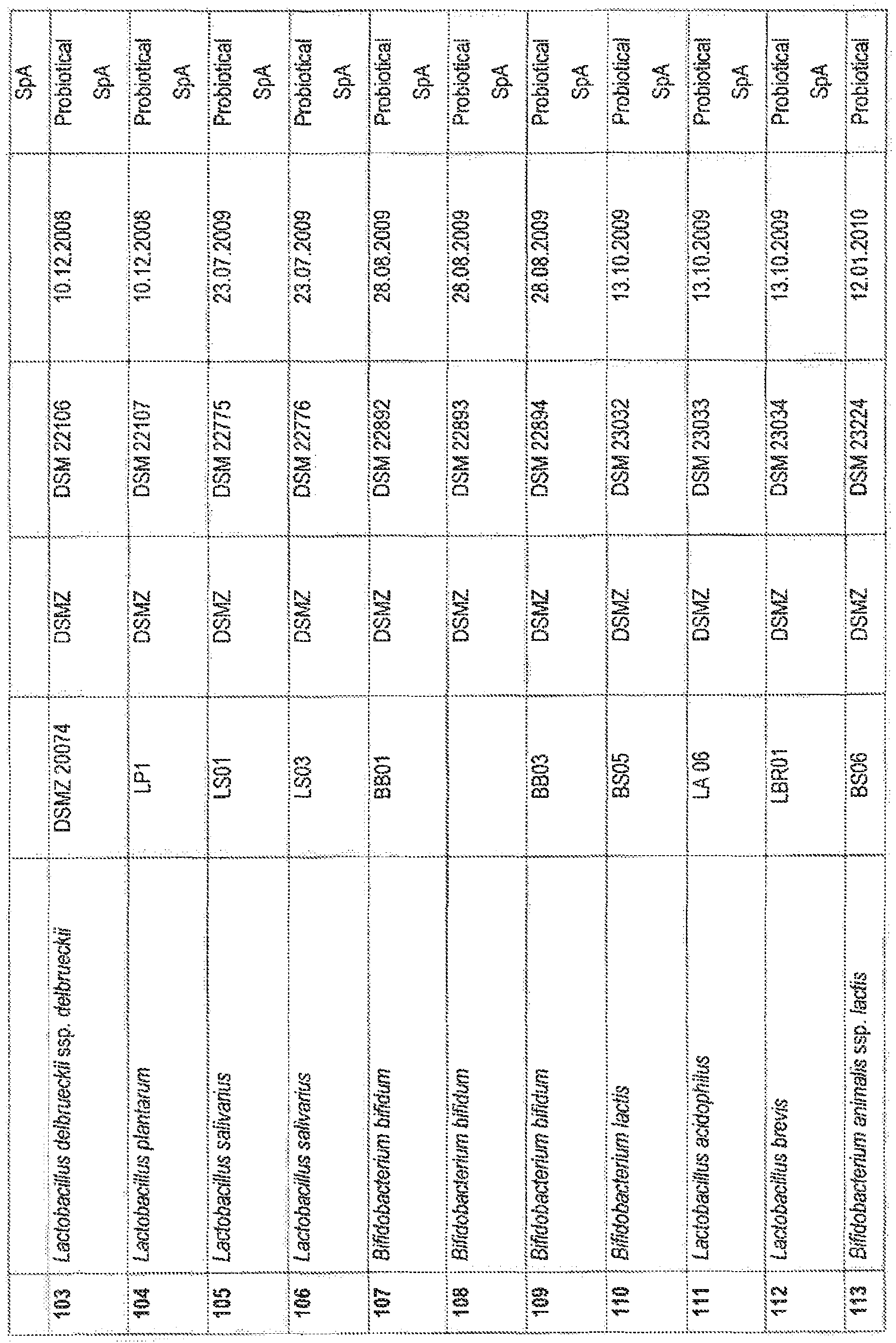

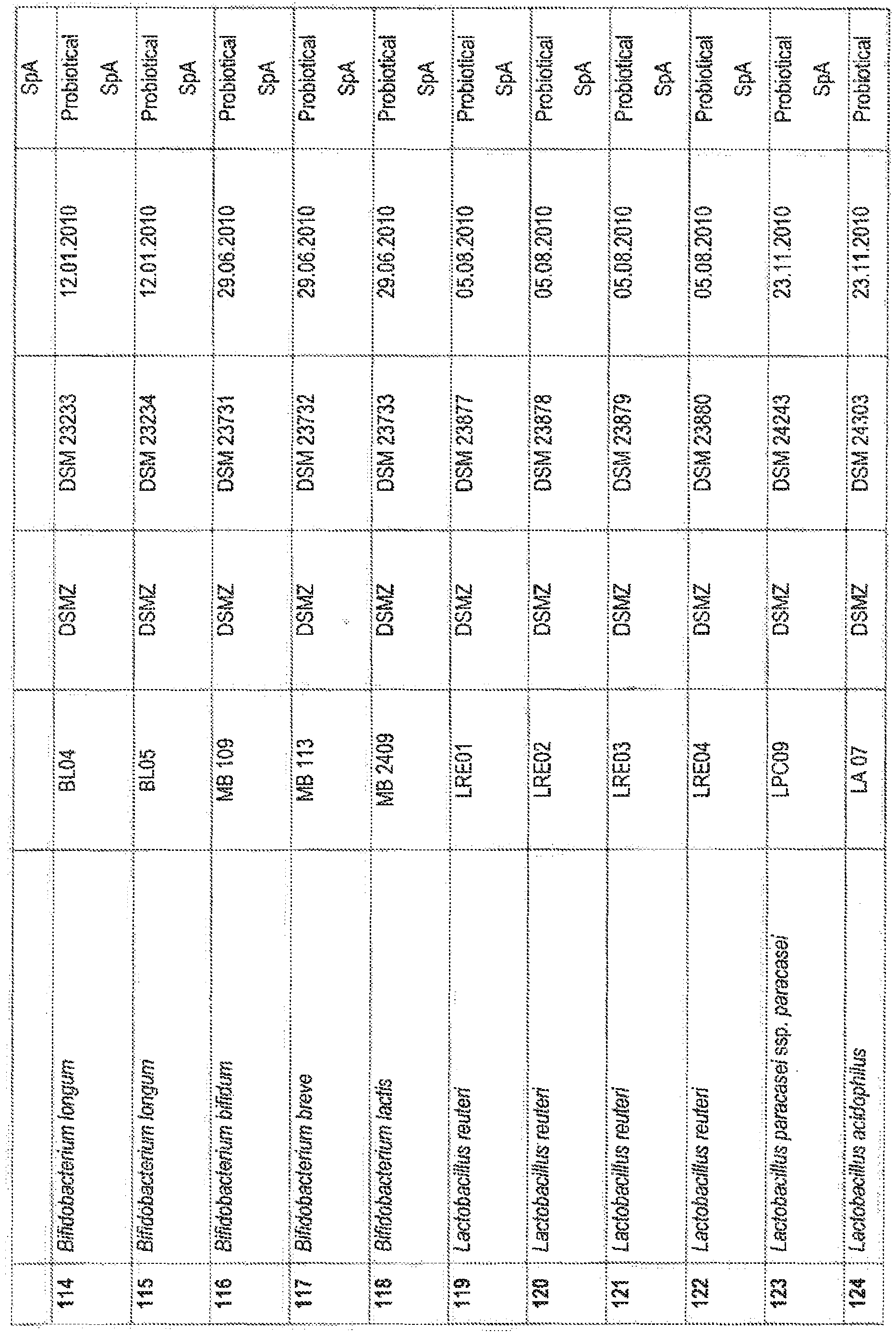

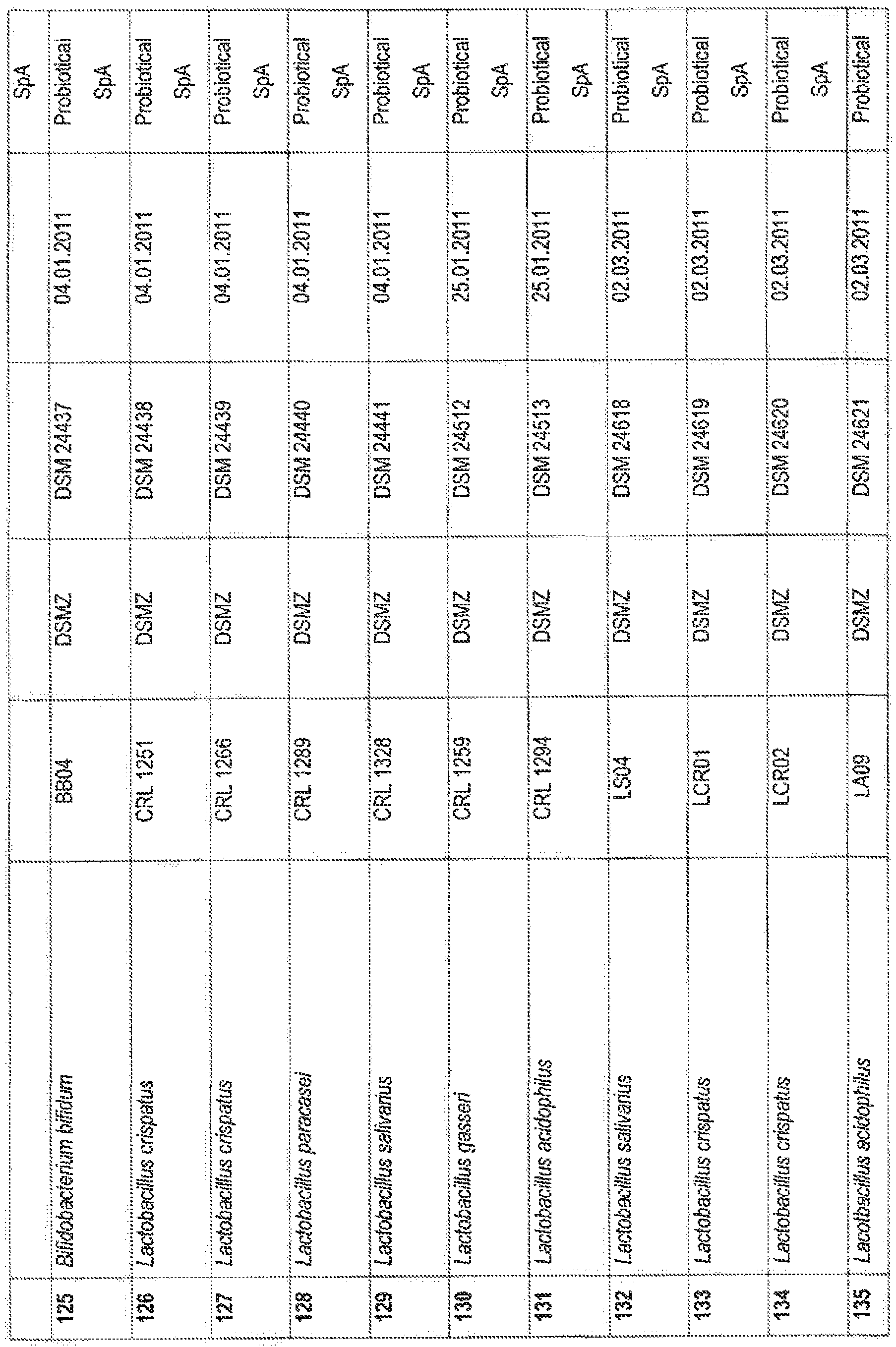

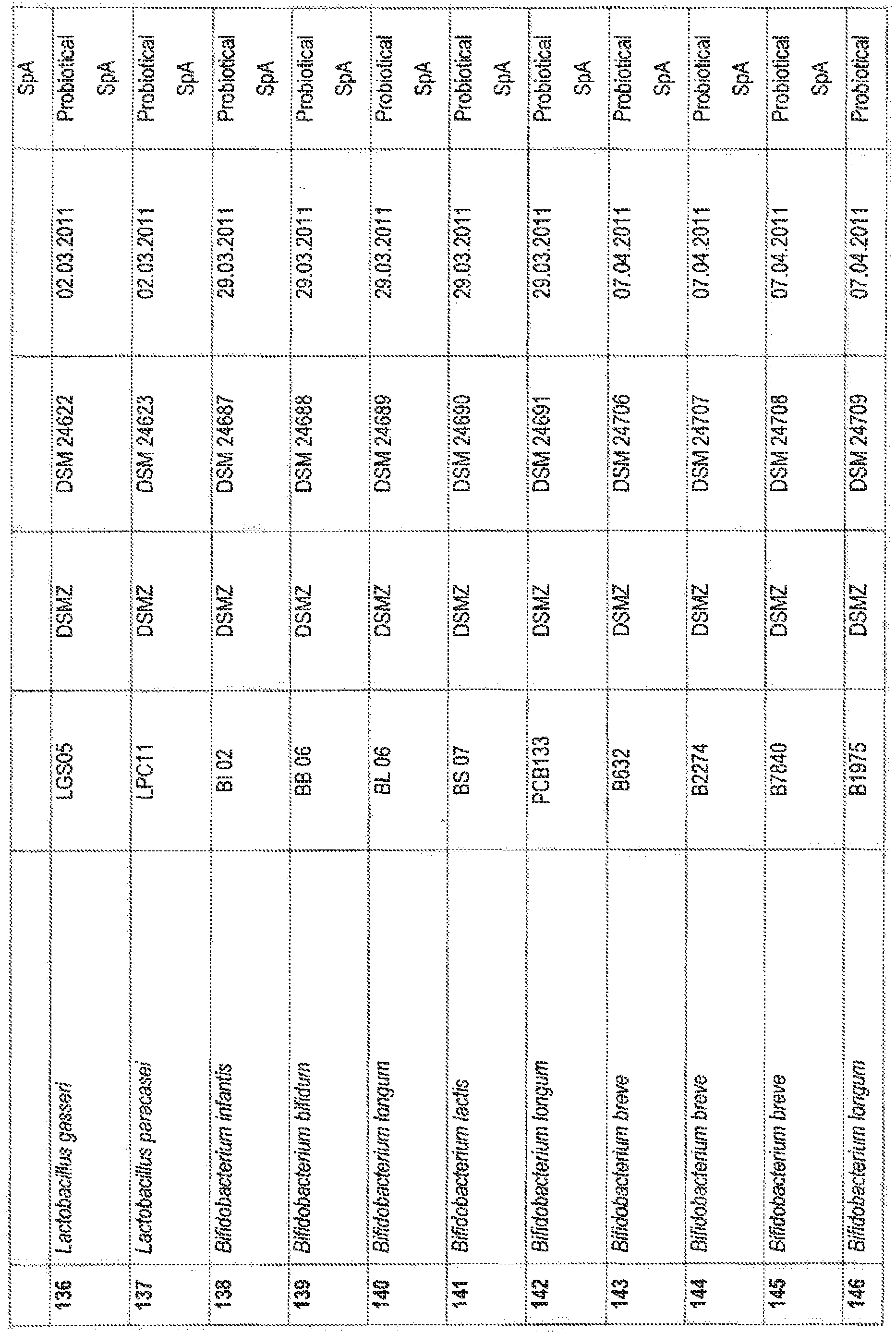

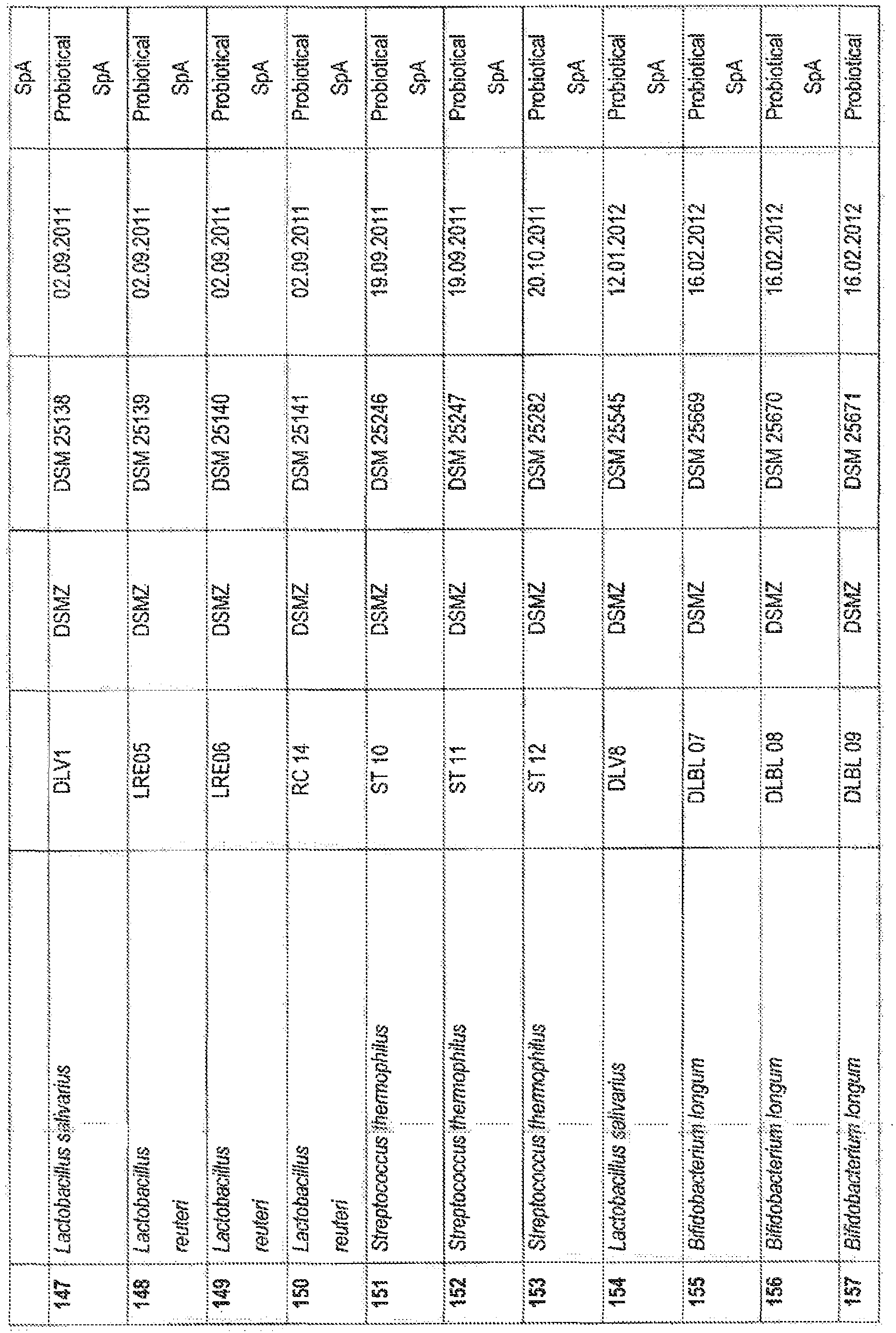

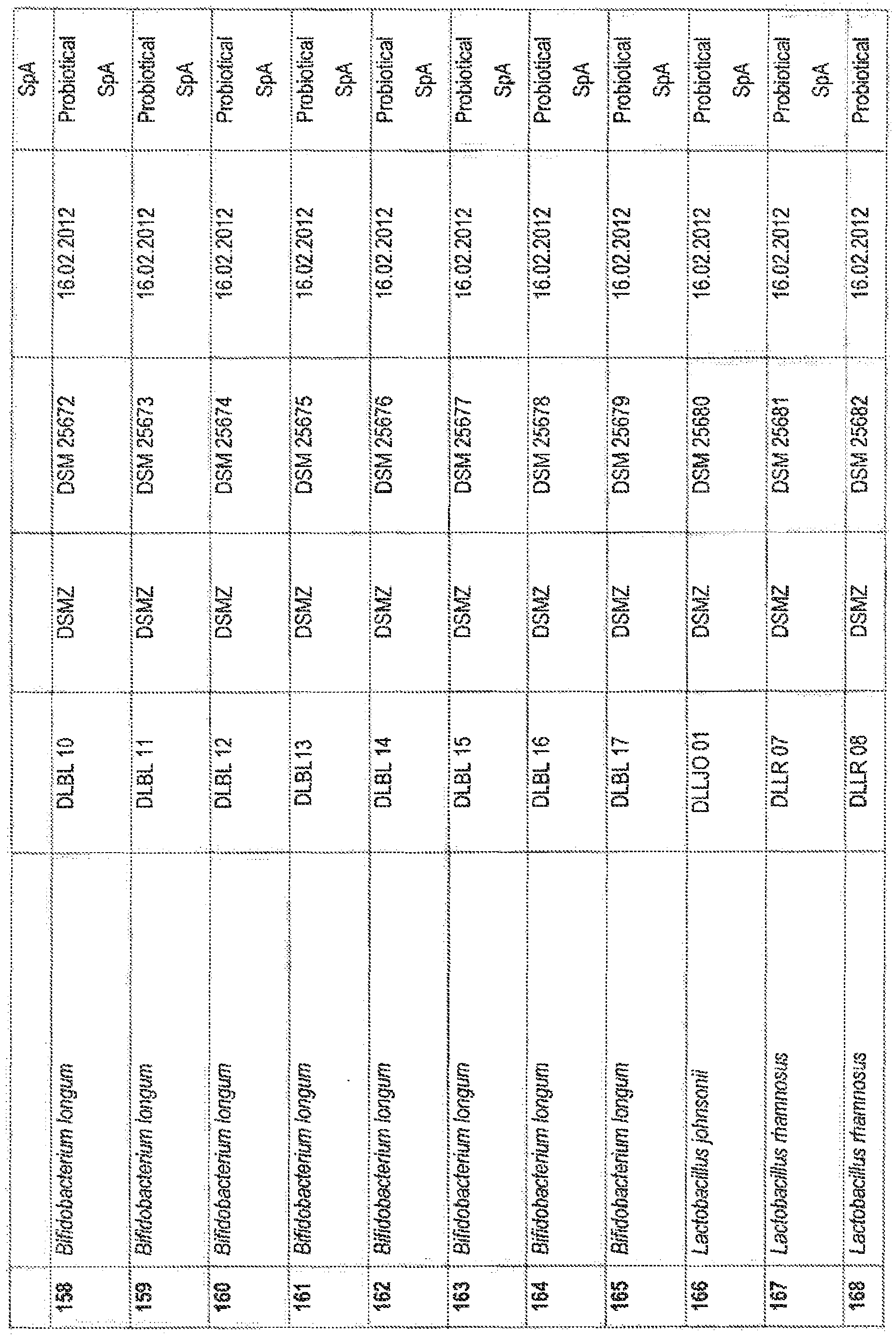

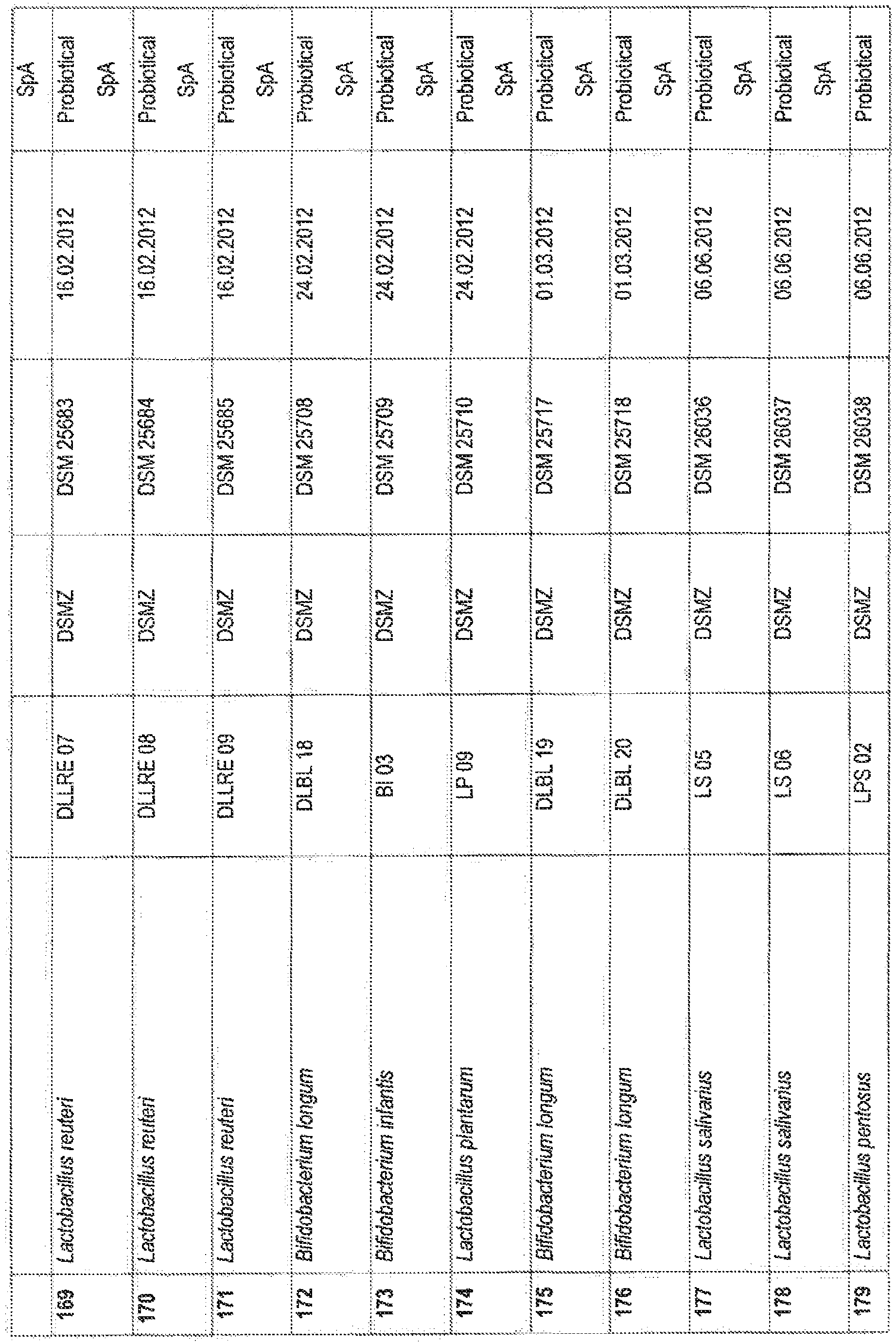

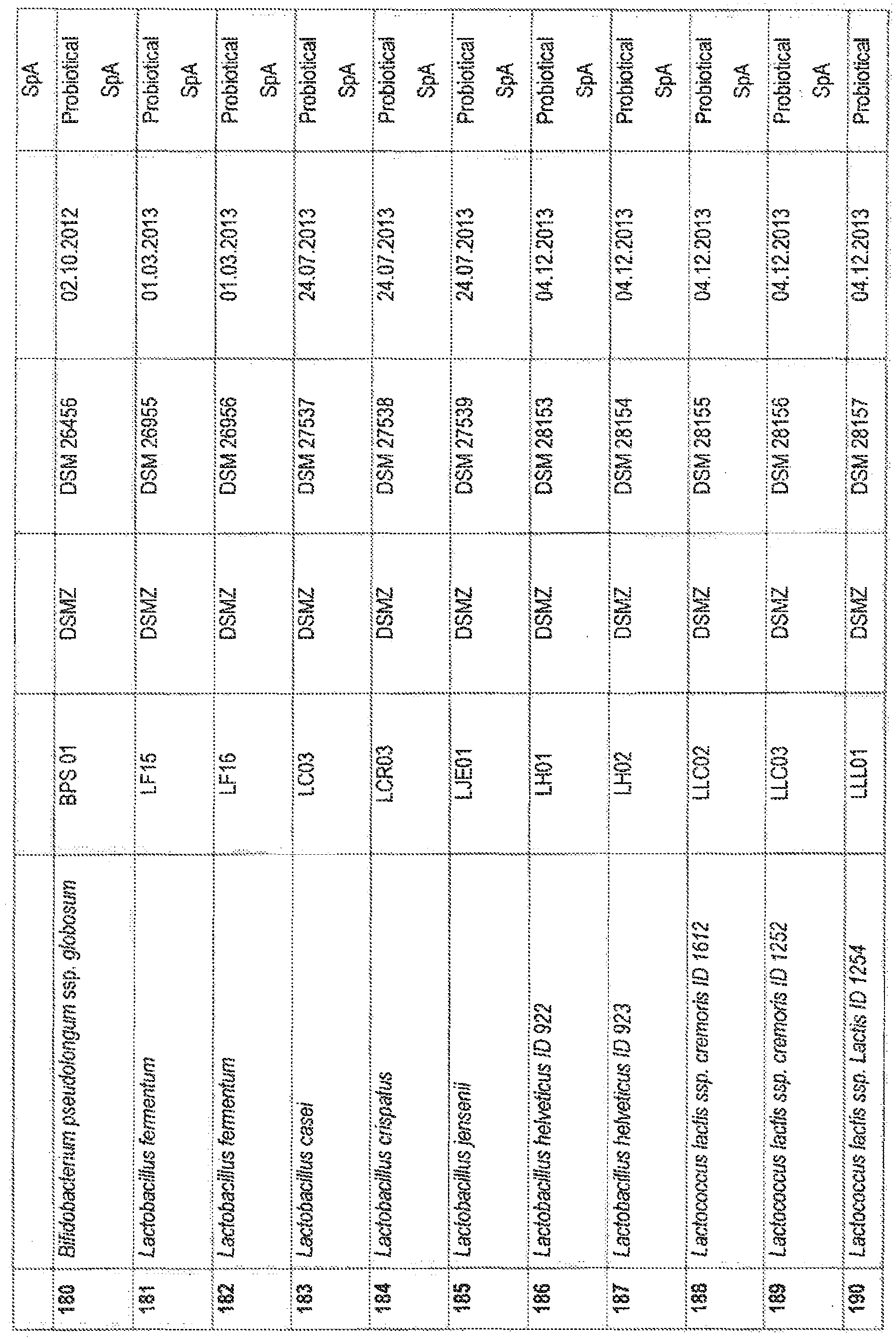

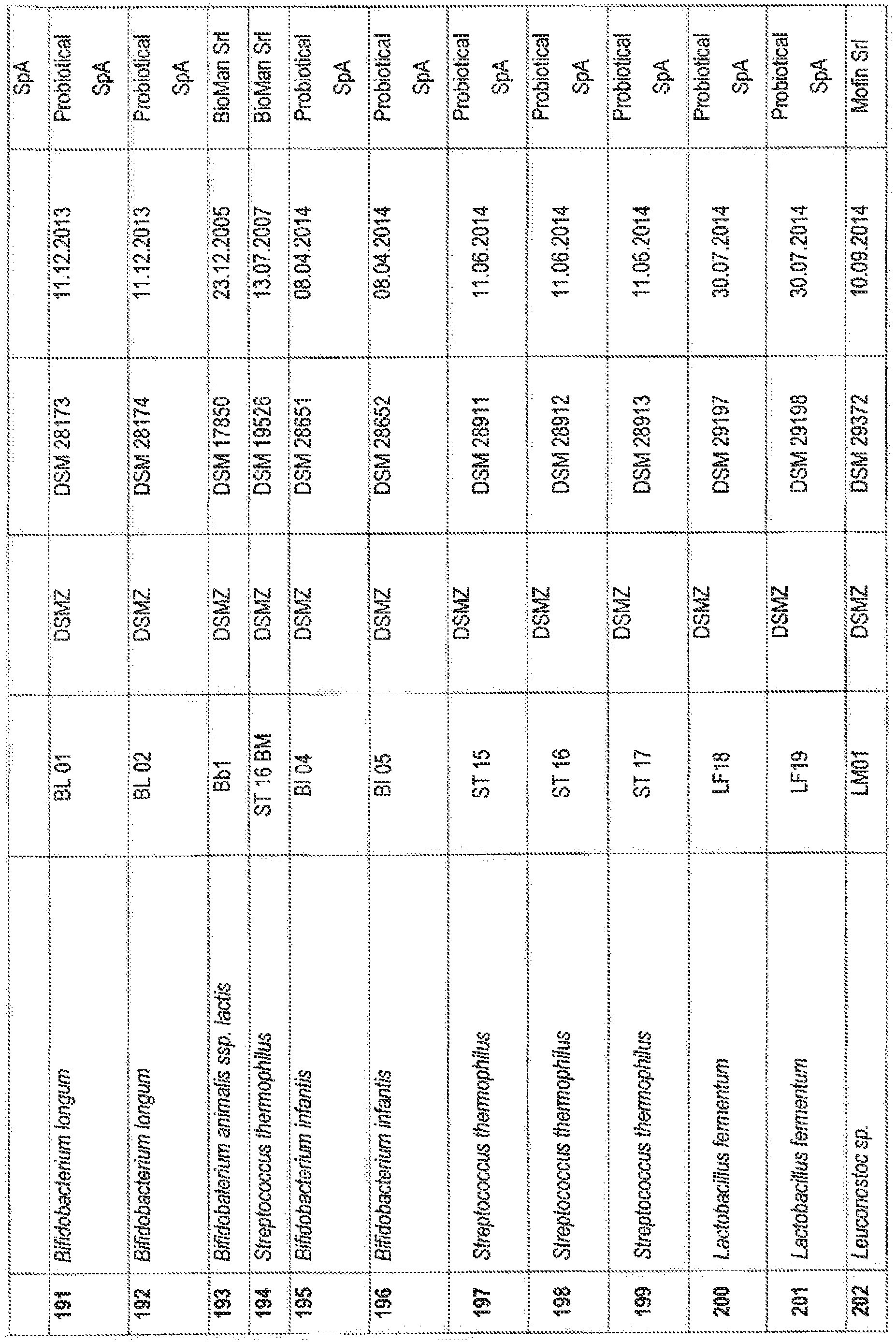

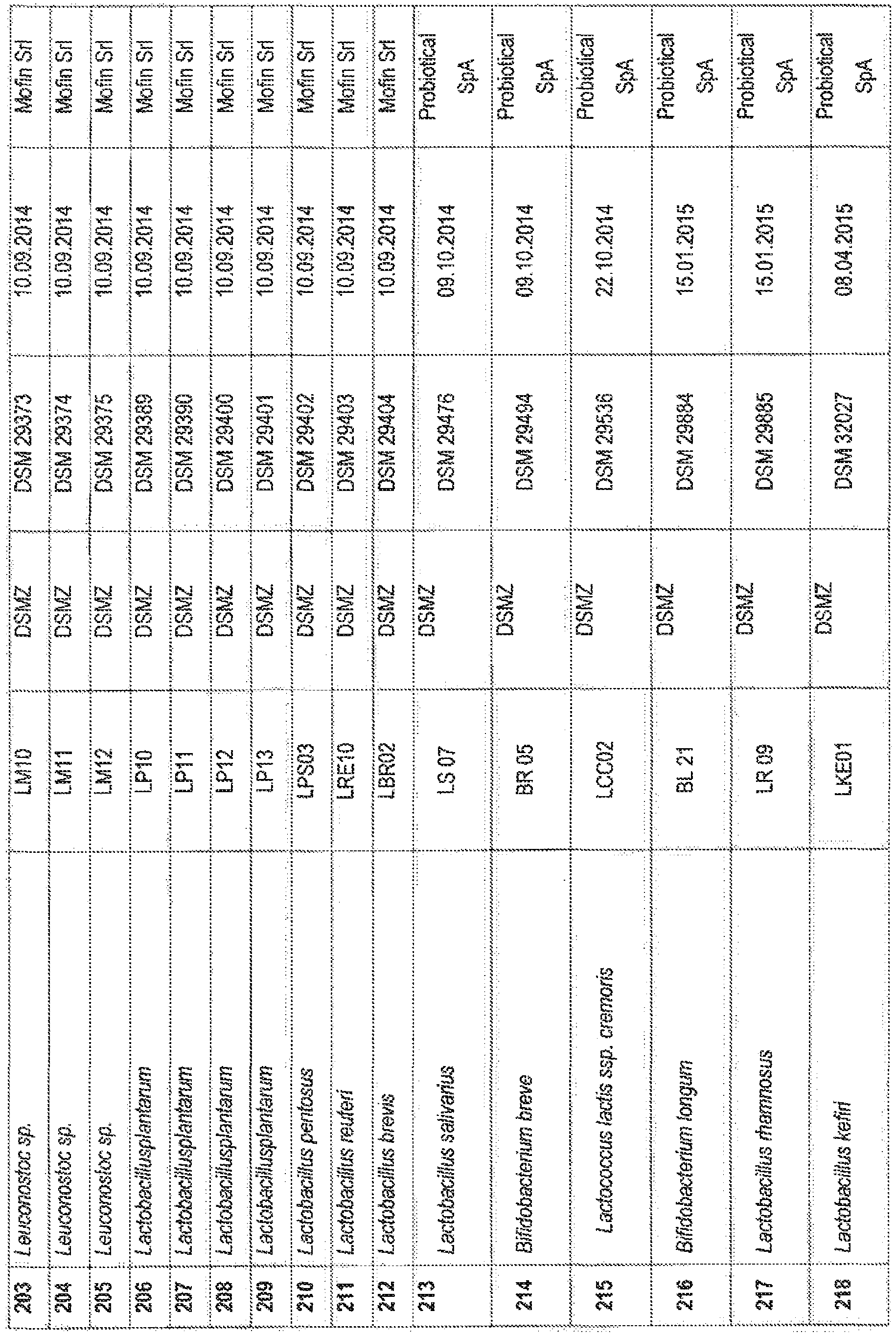

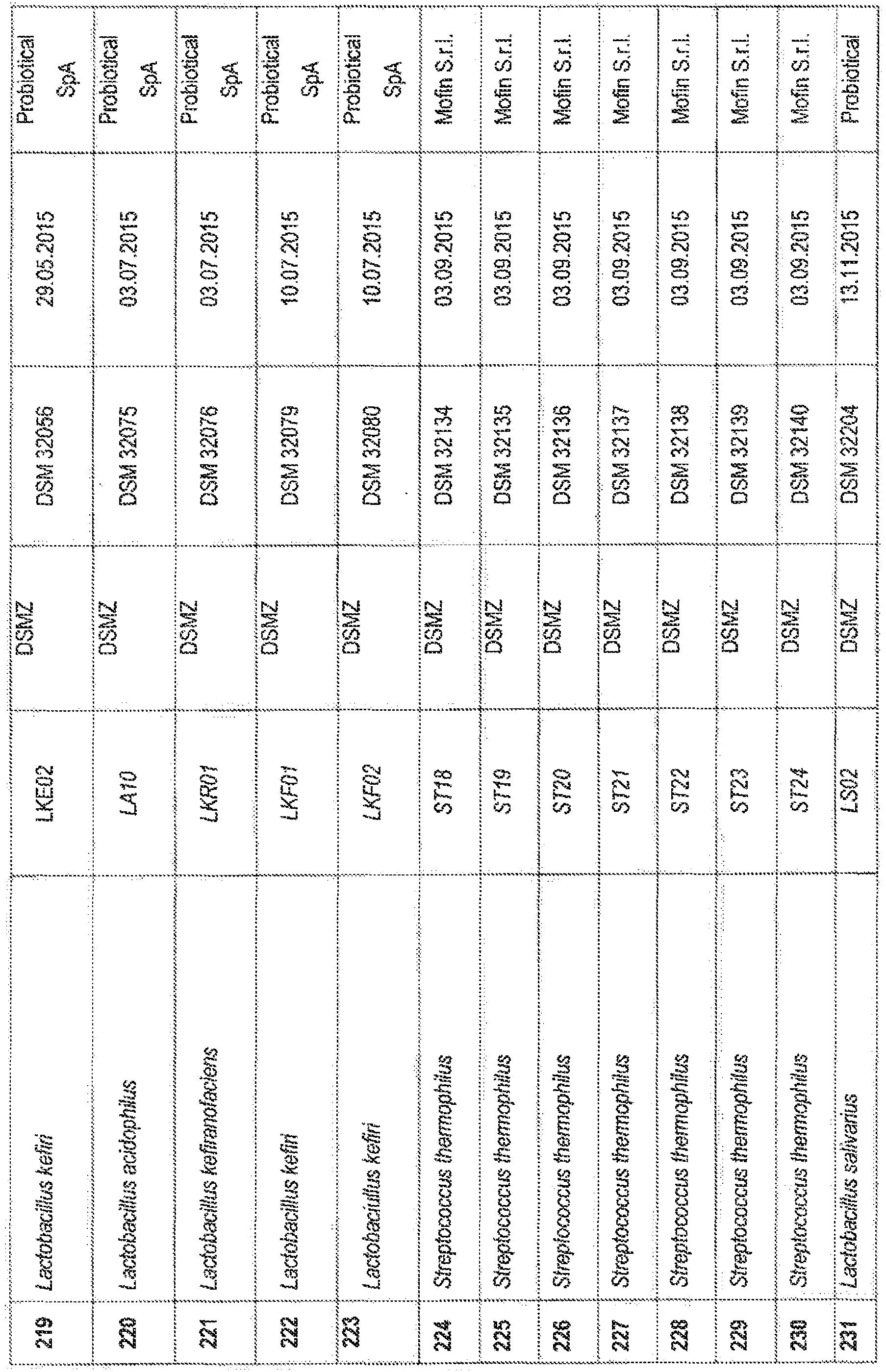

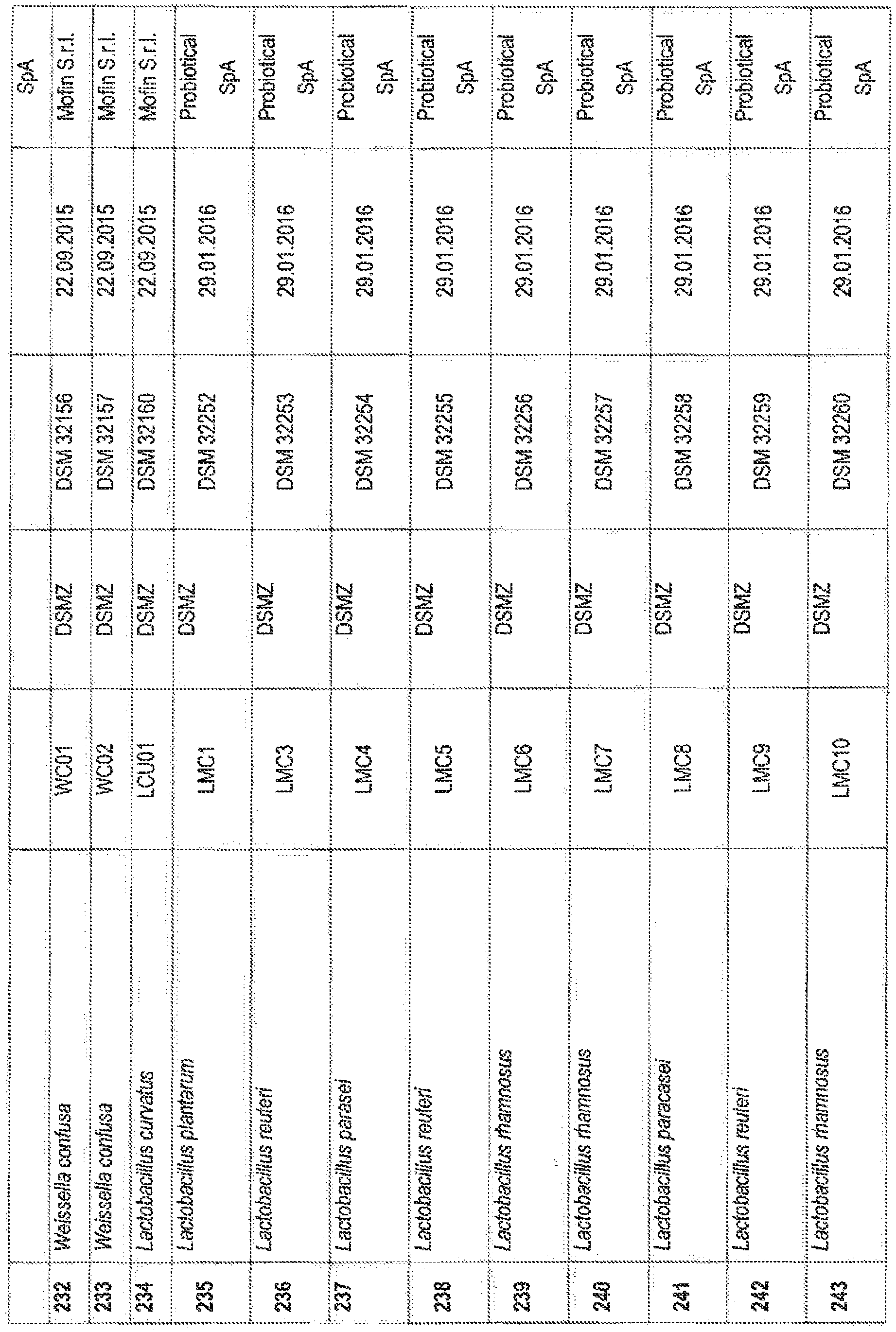

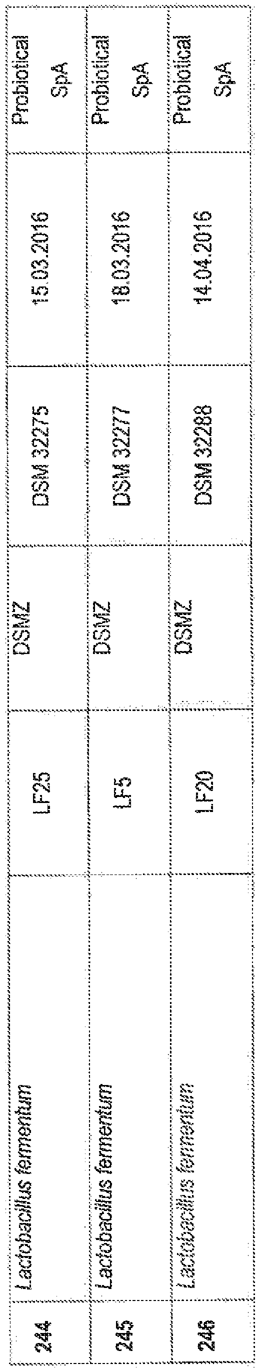

### Materials and Methods

### Experimental protocol

The experiment was conducted on a group of 6 volunteers, all female and aged between 29 and 41 years (mean age 34.5 years).

Each volunteer tested 5 formulations of melatonin (gel 0.1%, gel 0.2%, gel 1%, cream, Circadir 2 mg), with a washout period of one week after each application. Formulations of melatonin which are not as claimed are not according to the invention.

The saliva samples were taken according to the following experimental scheme:
h 10.00 Basal
h 10.30 30'
h 11.00 1h
h 13.00 3h
h 16.00 6h

The saliva was collected with Salivette (Sarstedt), following the instructions attached thereto (mouth rinsing before each sampling, chewing on the swab for 1 minute). The saliva was extracted from the swab by 2 min centrifugation at 4000 rpm, then divided into 300 µl aliquots and frozen at -20°C until the time of analysis.

### Data analysis

The melatonin assays were performed with the ELISA (Enzyme-Linked Immunosorbent Assay) technique, using the Melatonin (direct) Saliva kit (SLV-4779 - DRG Instruments, Germany). The analytical sensitivity and inter- and intra-assay coefficients of variation are the following: 0.3 pg/ml, 7.6 - 13.0 % and 6.1 - 10.8 %.

The kit in question is certified for in-vitro diagnostic use.

| **Patient ID** | **Gel 0.1% 1 mg/g** | | | | | |
|---|---|---|---|---|---|---|
| **1** | 0.42 | 6.65 | 7.91 | 18.2 | 14.6 | 37.76 |
| **2** | 0.33 | 4.88 | 4.22 | 4.72 | 2.33 | 16.48 |
| **3** | 0.1 | 1.6 | 1.62 | 7.55 | 2.09 | 10.94 |
| **4** | 1.93 | : 17.9 | 22.4 | 18.7 | 9.46 | 70.39 |
| **5** | 0.1 | 5.67 | 9.59 | 2.43 | 1.67 | 19.46 |
| **6** | 0.14 | 14.4 | 8.35 | 10.10 | 8.70 | 71.56 |
| | **Basal** | **30'** | **1h** | **3h** | **6h** | **total** |
| | | | | | Total 47.87 | 226.04 |
| | | | | | | mean=37.67 |

## Claims

1. A composition in the form of a gel for topical use comprising:
- an effective amount of an active substance, and
- a vehicle or carrier comprising water and a thickening viscous matrix comprising or, alternatively, consisting of a mixture of Poloxamer 188 and Poloxamer 407, wherein Poloxamer 188 is present in an amount comprised from 0.1% to 5% by weight, out of 100 g of composition, preferably in an amount comprised from 0.5% to 5% by weight or from 1% to 3% by weight; and Poloxamer 407 is present in an amount comprised from 1% to 30% by weight, out of 100 g of composition, preferably in an amount comprised from 5% to 30% by weight or from 10% to 20% by weight;
wherein the active substance is at least one microorganism selected from the group comprising or, alternatively, consisting of the strains: *Lactobacillus salivarius* (LS01) DSM 22775, deposited on 23.07.2009; *Bifidobacterium breve* (BR03) DSM 16604, deposited on 20.07.2004; *Lactobacillus pentosus* (LPS01) DSM 21980, deposited on 14.11.2008; *Streptococcus thermophilus* (FP4) DSM 18616, deposited on 13.09.2006; *Lactobacillus casei ssp. rhamnosus* (LR04) DSM 16605, deposited on 20.07.2004; and *Lactobacillus acidophilus* (LA02) DSM 21717, deposited on 06.08.2008; and a mixture thereof;
and
wherein said composition is for topical transdermal use in the preventive and/or curative treatment of pathologies, disorders or diseases associated with/deriving from alterations of the immune system selected from the group comprising allergies, atopy, food hypersensitivity, dermatitis, atopic dermatitis, eczema and psoriasis.

2. The composition for use according to claim 1, wherein said at least one microorganism is selected from the group comprising or, alternatively, consisting of the strains: *Lactobacillus salivarius* (LS01) DSM 22775, *Bifidobacterium breve* (BR03) DSM 16604, *Lactobacillus pentosus* (LPS01) DSM 21980 and a mixture thereof.

3. The composition for use in accordance with one any one of the preceding claims, further comprising, as an active substance, an effective amount of at least one compound selected from the group comprising or, alternatively, consisting of: rutin, rutoxides, arbutin, oxerutin, diosmin, hesperidin and troxerutin, arctigenin, arctin, berberine, berbamine, sanguinarine and chelerythrine, as such or in the form of plant extracts containing said compounds, on their own.

4. The composition for use in accordance with any one of the preceding claims, wherein:
- said at least one microorganism is present at a concentration comprised from 1x10⁶ CFU/g to 1x10¹² CFU/g of composition, and wherein:
- said microorganisms are present in said composition in an amount by weight comprised from 0.1% to 5%, relative to the total weight of composition.

5. The composition for use in accordance with claim 4, wherein:
- said at least one microorganism is present at a concentration comprised from 1x10⁷ CFU/g to 1x10¹¹ CFU/g of composition, preferably from
1x10⁸ CFU/g to 1x10¹⁰ CFU/g of composition, and wherein:
- said microorganisms are present in said composition in an amount by weight comprised from 0.5% to 3%, preferably in an amount by weight comprised from 1% to 2%, relative to the total weight of composition.

6. The composition for use in accordance with any one of the preceding claims, wherein:
said composition further comprises an effective amount of an active substance selected from:
- at least one compound selected from the group comprising or, alternatively, consisting of rutin, rutoxides, arbutin, oxerutin, diosmin, hesperidin and troxerutin, arctigenin, arctin, berberine, berbamine, sanguinarine and chelerythrine, as such or in the form of plant extracts containing said compounds, on their own; and/or
- melatonin;
said active substance being present in said composition in an amount by weight comprised from 0.1% to 10% by weight, preferably in an amount comprised from 0.5% to 5% by weight, even more preferably from 1% to 3% by weight, relative to the total weight of the composition.

7. The composition for use in accordance with any one of the preceding claims, wherein said composition further comprises additives and/or excipients and/or adjuvants selected from among those capable of promoting the formation and stabilization of the gel, selected from the group comprising or, alternatively, consisting of acid salts, sodium sorbate, potassium sorbate, sodium benzoate, potassium benzoate, glycols, ethylene glycol and propylene glycol.

8. A process for the preparation of a composition for use in accordance with any one of the preceding claims, said process comprising the following steps:
- mixing in water, under continuous stirring, the various components, added in succession one after the other or separately premixed and added together, using a mixer provided with a temperature control and adjustment means and a stirring means, wherein the temperature is set at values lower than room temperature, comprised from 2°C to 16°C, preferably from 4°C to 10°C, for example from 6°C to 8°C, for a time comprised from 1 minute to 15 minutes so as to obtain a homogeneous mixture;
- maintaining said homogeneous mixture, preferably under continuous stirring, at a temperature comprised from 2°C to 10°C, preferably at a temperature comprised from 4°C to 8°C, for example from 5°C to 7°C, for a time comprised from 1 minute to 60 minutes, preferably from 5 minutes to 40 minutes, even more preferably from 10 minutes to 20 minutes, so as to obtain said composition in a liquid state;
- packaging said composition in sealed containers.

## Patentansprüche

1. Zusammensetzung in Form eines Gels zur topischen Anwendung, umfassend:
- eine wirksame Menge eines Wirkstoffs, und
- ein Vehikel oder einen Träger, umfassend Wasser und eine verdickende viskose Matrix, umfassend oder alternativ bestehend aus einer Mischung aus Poloxamer 188 und Poloxamer 407, wobei Poloxamer 188 in einer Menge von 0,1 bis 5 Gew.- % von 100 g der Zusammensetzung, vorzugsweise in einer Menge von 0,5 bis 5 Gew.- % oder von 1 bis 3 Gew.- %, vorhanden ist; und Poloxamer 407 in einer Menge von 1 bis 30 Gew.- % von 100 g der Zusammensetzung, vorzugsweise in einer Menge von 5 bis 30 Gew.- % oder von 10 bis 20 Gew.- %, vorhanden ist;
wobei der Wirkstoff mindestens ein Mikroorganismus ist, aus der Gruppe ausgewählt, umfassend oder alternativ bestehend aus den Stämmen: *Lactobacillus salivarius* (LS01) DSM 22775, hinterlegt am 23.07.2009; *Bifidobacterium breve* (BR03) DSM 16604, hinterlegt am 20.07.2004; *Lactobacillus pentosus* (LPS01) DSM 21980, hinterlegt am *14.11.2008; Streptococcus thermophilus* (FP4) DSM 18616, hinterlegt am 13.09.2006; *Lactobacillus casei ssp. rhamnosus* (LR04) DSM 16605, hinterlegt am 20.07.2004; und *Lactobacillus acidophilus* (LA02) DSM 21717, hinterlegt am 06.08.2008; und eine Mischung davon;
und
wobei die Zusammensetzung zur topischen transdermalen Verwendung bei der präventiven und/oder kurativen Behandlung von Pathologien, Störungen oder Krankheiten bestimmt ist, die mit Veränderungen des Immunsystems verbunden/aus ihnen hergeleitet sind, die aus der Gruppe ausgewählt sind, die Allergien, Atopie, Nahrungsmittelüberempfindlichkeit, Dermatitis, atopische Dermatitis, Ekzem und Psoriasis umfasst.

2. Zusammensetzung zur Verwendung nach Anspruch 1, wobei der mindestens ein Mikroorganismus aus der Gruppe ausgewählt ist, umfassend oder alternativ bestehend aus den Stämmen: *Lactobacillus salivarius* (LS01) DSM 22775, *Bifidobacterium breve* (BR03) DSM 16604, *Lactobacillus pentosus* (LPS01) DSM 21980 und eine Mischung davon.

3. Zusammensetzung zur Verwendung nach einem der vorhergehenden Ansprüche, ferner umfassend als Wirkstoff eine wirksame Menge mindestens einer Verbindung, aus der Gruppe ausgewählt, umfassend oder alternativ bestehend aus: Rutin, Rutoxiden, Arbutin, Oxerutin, Diosmin, Hesperidin und Troxerutin, Arctigenin, Arctin, Berberin, Berbamin, Sanguinarin und Chelerythrin, als solche oder in Form von Pflanzenextrakten, die diese Verbindungen enthalten, allein.

4. Zusammensetzung zur Verwendung nach einem der vorhergehenden Ansprüche, wobei:
- der mindestens ein Mikroorganismus in einer Konzentration von 1x10⁶ KBE/g bis 1x10¹² KBE/g der Zusammensetzung vorhanden ist, und wobei:
- die Mikroorganismen in der Zusammensetzung in einer Gewichtsmenge von 0,1 % bis 5 %, bezogen auf das Gesamtgewicht der Zusammensetzung, vorhanden sind.

5. Zusammensetzung zur Verwendung nach Anspruch 4, wobei:
- der mindestens ein Mikroorganismus in einer Konzentration von 1x10⁷ KBE/g bis 1x10¹¹ KBE/g der Zusammensetzung, vorzugsweise von 1x10⁸ KBE/g bis 1x10¹⁰ KBE/g der Zusammensetzung, vorhanden ist, und wobei:
- die Mikroorganismen in der Zusammensetzung in einer Gewichtsmenge von 0,5 % bis 3 %, vorzugsweise in einer Gewichtsmenge von 1 % bis 2 %, bezogen auf das Gesamtgewicht der Zusammensetzung, vorhanden sind.

6. Zusammensetzung zur Verwendung nach einem der vorhergehenden Ansprüche, wobei:
die Zusammensetzung ferner eine wirksame Menge eines Wirkstoffs umfasst, ausgewählt aus:
- mindestens einer Verbindung aus der Gruppe ausgewählt, umfassend oder alternativ bestehend aus Rutin, Rutoxiden, Arbutin, Oxerutin, Diosmin, Hesperidin und Troxerutin, Arctigenin, Arctin, Berberin, Berbamin, Sanguinarin und Chelerythrin, als solche oder in Form von Pflanzenextrakten, die diese Verbindungen enthalten, allein; und/oder
- Melatonin;
wobei der Wirkstoff in der Zusammensetzung in einer Gewichtsmenge von 0,1 bis 10 Gew.- %, vorzugsweise in einer Menge von 0,5 bis 5 Gew.- %, noch bevorzugter von 1 bis 3 Gew.- %, bezogen auf das Gesamtgewicht der Zusammensetzung, vorhanden ist.

7. Zusammensetzung zur Verwendung nach einem der vorhergehenden Ansprüche, wobei die Zusammensetzung ferner Additive und/oder Hilfsstoffe und/oder Adjuvantien umfasst, die aus denjenigen ausgewählt sind, die in der Lage sind, die Bildung und Stabilisierung des Gels zu fördern, die aus der Gruppe ausgewählt sind, die saure Salze, Natriumsorbat, Kaliumsorbat, Natriumbenzoat, Kaliumbenzoat, Glykole, Ethylenglykol und Propylenglykol umfasst oder alternativ daraus besteht.

8. Verfahren zur Herstellung einer Zusammensetzung zur Verwendung nach einem der vorhergehenden Ansprüche, wobei das Verfahren die folgenden Schritte umfasst:
- in Wasser mischen, unter ständigem Rühren, der verschiedenen Komponenten, die in Folge eine nach der anderen oder getrennt vorgemischt und zusammengegeben werden, unter Verwendung eines Mischers, der mit Temperatursteuerungs- und - einstellmitteln und Rührmitteln versehen ist, wobei die Temperatur auf Werte niedriger als Raumtemperatur eingestellt wird, die im Bereich von 2 °C bis 16 °C, vorzugsweise von 4 °C bis 10 °C, beispielsweise von 6 °C bis 8 °C, für einen Zeitraum von 1 Minute bis 15 Minuten liegen, um eine homogene Mischung zu erhalten;
- halten der homogenen Mischung, vorzugsweise unter ständigem Rühren, bei einer Temperatur von 2 °C bis 10 °C, vorzugsweise bei einer Temperatur von 4 °C bis 8 °C, beispielsweise von 5 °C bis 7 °C, für einen Zeitraum von 1 Minute bis 60 Minuten, vorzugsweise von 5 Minuten bis 40 Minuten, noch bevorzugter von 10 Minuten bis 20 Minuten, um die Zusammensetzung in einem flüssigen Zustand zu erhalten;
- verpacken der Zusammensetzung in versiegelten Behältern.

## Revendications

1. Composition sous forme de gel à usage topique, comprenant :
- une quantité efficace d'une substance active, et
- un véhicule ou support comprenant de l'eau et une matrice visqueuse épaississante comprenant ou, en variante, constituée d'un mélange de Poloxamer 188 et de Poloxamer 407, dans laquelle le Poloxamer 188 est présent dans une quantité comprise de 0,1 à 5 % en poids, pour 100 g de composition, de préférence dans une quantité comprise de 0,5 à 5 % en poids ou de 1 à 3 % en poids ; et le Poloxamer 407 est présent dans une quantité comprise de 1 à 30 % en poids, pour 100 g de composition, de préférence dans une quantité comprise de 5 à 30 % en poids ou de 10 à 20 % en poids ;
dans laquelle la substance active est au moins un micro-organisme choisi dans le groupe comprenant ou, en variante, constitué des souches suivantes : *Lactobacillus salivarius* (LS01) DSM 22775, déposée le 23/07/2009 ; *Bifidobacterium breve* (BR03) DSM 16604, déposée le 20/07/2004 ; *Lactobacillus pentosus* (LPS01) DSM 21980, déposée le 14/11/2008 ; *Streptococcus thermophilus* (FP4) DSM 18616, déposée le 13/09/2006 ; *Lactobacillus casei ssp. rhamnosus* (LR04) DSM 16605, déposée le 20/07/2004 ; et *Lactobacillus acidophilus* (LA02) DSM 21717, déposée le 06/08/2008 ; et un mélange de celles-ci ;
et
dans laquelle ladite composition est destinée à un usage topique transdermique dans le traitement préventif et/ou curatif de pathologies, de troubles ou de maladies associés à des/découlant d'altérations du système immunitaire, choisis dans le groupe comprenant les allergies, l'atopie, l'hypersensibilité alimentaire, la dermatite, la dermatite atopique, l'eczéma et le psoriasis.

2. Composition destinée à être utilisée selon la revendication 1, dans laquelle ledit au moins un micro-organisme est choisi dans le groupe comprenant ou, en variante, constitué des souches suivantes : *Lactobacillus salivarius* (LS01) DSM 22775, *Bifidobacterium breve* (BR03) DSM 16604, *Lactobacillus pentosus* (LPS01) DSM 21980 et un mélange de celles-ci.

3. Composition destinée à être utilisée selon l'une quelconque des revendications précédentes, comprenant en outre, en tant que substance active, une quantité efficace d'au moins un composé choisi dans le groupe comprenant ou, en variante, constitué de : la rutine, les rutoxides, l'arbutine, l'oxerutine, la diosmine, l'hespéridine et la troxérutine, l'arctigénine, l'arctine, la berbérine, la berbamine, la sanguinarine et la chélérythrine, telles quelles ou sous forme d'extraits végétaux contenant lesdits composés, seuls.

4. Composition destinée à être utilisée selon l'une quelconque des revendications précédentes, dans laquelle :
- ledit au moins un micro-organisme est présent à une concentration comprise de 1 x 10⁶ UFC/g à 1 x 10¹² UFC/g de composition, et dans laquelle :
- lesdits micro-organismes sont présents dans ladite composition dans une quantité en poids comprise de 0,1 à 5 %, par rapport au poids total de la composition.

5. Composition destinée à être utilisée selon la revendication 4, dans laquelle :
- ledit au moins un micro-organisme est présent à une concentration comprise de 1 x 10⁷ UFC/g à 1 x 10¹¹ UFC/g de composition, de préférence de
1 x 10⁸ UFC/g à 1 x 10¹⁰ UFC/g de composition, et dans laquelle :
- lesdits micro-organismes sont présents dans ladite composition dans une quantité en poids comprise de 0,5 à 3 %, de préférence dans une quantité en poids comprise de 1 à 2 %, par rapport au poids total de la composition.

6. Composition destinée à être utilisée selon l'une quelconque des revendications précédentes, dans laquelle :
ladite composition comprend en outre une quantité efficace d'une substance active choisie parmi :
- au moins un composé choisi dans le groupe comprenant ou, en variante, constitué de la rutine, les rutoxides, l'arbutine, l'oxerutine, la diosmine, l'hespéridine et la troxérutine, l'arctigénine, l'arctine, la berbérine, la berbamine, la sanguinarine et la chélérythrine, telles quelles ou sous forme d'extraits végétaux contenant lesdits composés, seuls ;
et/ou
- de la mélatonine ;
ladite substance active étant présente dans ladite composition dans une quantité en poids comprise de 0,1 à 10 % en poids, de préférence dans une quantité comprise de 0,5 à 5 % en poids, et plus préférablement encore de 1 à 3 % en poids, par rapport au poids total de la composition.

7. Composition destinée à être utilisée selon l'une quelconque des revendications précédentes, dans laquelle ladite composition comprend en outre des additifs et/ou des excipients et/ou des adjuvants choisis parmi ceux capables de favoriser la formation et la stabilisation du gel, choisis dans le groupe comprenant ou, en variante, constitué de sels acides, de sorbate de sodium, de sorbate de potassium, de benzoate de sodium, de benzoate de potassium, de glycols, d'éthylène glycol et de propylène glycol.

8. Procédé de préparation d'une composition destinée à être utilisée selon l'une quelconque des revendications précédentes, ledit procédé comprenant les étapes suivantes :
- mélanger dans de l'eau, sous agitation continue, les différents composants, ajoutés successivement les uns après les autres ou prémélangés séparément puis ajoutés ensemble, à l'aide d'un mélangeur pourvu de moyens de contrôle et de réglage de la température et de moyens d'agitation, dans lequel la température est réglée à des valeurs inférieures à la température ambiante, comprises de 2 à 16 °C, de préférence de 4 à 10 °C, par exemple de 6 à 8 °C, pendant une durée comprise de 1 minute à 15 minutes de manière à obtenir un mélange homogène ;
- maintenir ledit mélange homogène, de préférence sous agitation continue, à une température comprise de 2 à 10 °C, de préférence à une température comprise de 4 à 8 °C, par exemple de 5 à 7 °C, pendant une durée comprise de 1 minute à 60 minutes, de préférence de 5 minutes à 40 minutes, et plus préférablement encore de 10 minutes à 20 minutes, de manière à obtenir ladite composition à l'état liquide ;
- conditionner ladite composition dans des récipients étanches.
